# FASCICULE DE BREVET EUROPEEN

(11) **EP 1 299 532 B1**
(45) Date de publication et mention de la délivrance du brevet: **09.04.2008**
(21) Numéro de dépôt: 01945414.9
(22) Date de dépôt: 13.06.2001
(51) Int. Cl.: C12N 15/10, C12N 15/64, C12N 9/02, C12Q 1/68

(54) **BANQUES COMBINATOIRES AMELIOREES PAR RECOMBINAISON DANS LA LEVURE ET PROCEDE D'ANALYSE**
VERBESSERTE KOMBINATORISCHE GENBANKEN DURCH REKOMBINATION IN HEFE UND ANALYSEVERFAHREN
IMPROVED COMBINATORIAL LIBRARIES BY RECOMBINATION IN YEAST AND ANALYSIS METHOD

(30) Priorité: 14.06.2000 FR 0007555
(43) Date de publication de la demande: 09.04.2003
(73) Titulaire: Aventis Pharma S.A., 92160 Antony (FR); CENTRE NATIONAL DE LA RECHERCHE SCIENTIFIQUE (CNRS), 75794 Paris Cedex 16 (FR)
(72) Inventeur: TRUAN, Gilles, F-91340 Ollainville (FR); ABECASSIS, Valérie, F-91140 Villebon-Sur-Yvette (FR); POMPON, Denis, 91190 Gif-Sur-Yvette (FR)
(74) Mandataire: Bouvet, Philippe
(86) Numéro de dépôt international: PCT/FR2001/001831
(87) Numéro de publication internationale: WO 2001/096555

(56) Documents cités:
- EP-A- 0 934 999
- WO-A-00/42560
- WO-A-97/10344
- WO-A-99/40208
- WO-A-99/60096
- ABECASSIS V ET AL: "High efficiency family shuffling based on multi-step PCR and in vivo DNA recombination in yeast: statistical and functional analysis of a combinatorial library between human cytochrome P450 1A1 and 1A2." NUCLEIC ACIDS RESEARCH, (2000 OCT 15) 28 (20) E88. JOURNAL CODE: O8L;0411011. , 15 octobre 2000 (2000-10-15), XP002179392
- BELLAMINE A ET AL: "CHIMERAS OF THE HUMAN CYTOCHROME P450 1A FAMILY PRODUCED IN YEAST A COMMUNICATION IN MICROSOMAL MEMBRANES, ENZYME KINETICS AND STABILITY" EUROPEAN JOURNAL OF BIOCHEMISTRY, BERLIN, DE, vol. 225, no. 3, 1994, pages 1005-1013, XP000982831 ISSN: 0014-2956 cité dans la demande
- POMPON D ET AL: "PROTEIN ENGINEERING BY CDNA RECOMBINATION IN YEASTS: SHUFFLING OF MAMMALIAN CYTOCHROME P-450 FUNCTIONS" GENE, ELSEVIER BIOMEDICAL PRESS. AMSTERDAM, NL, vol. 83, no. 1, 15 novembre 1989 (1989-11-15), pages 15-24, XP000054542 ISSN: 0378-1119
- HANSSON L O ET AL: "Evolution of differential substrate specificities in Mu class glutathione transferases probed by DNA shuffling" JOURNAL OF MOLECULAR BIOLOGY,LONDON,GB, vol. 287, no. 2, 26 mars 1999 (1999-03-26), pages 265-276, XP002154179 ISSN: 0022-2836
- KIKUCHI M ET AL: "Novel family shuffling methods for the in vitro evolution of enzymes" GENE,NL,ELSEVIER BIOMEDICAL PRESS. AMSTERDAM, vol. 236, no. 1, 5 août 1999 (1999-08-05), pages 159-167, XP004175459 ISSN: 0378-1119 cité dans la demande
- KIKUCHI M ET AL: "An effective family shuffling method using single-stranded DNA" GENE,NL,ELSEVIER BIOMEDICAL PRESS. AMSTERDAM, vol. 243, no. 1-2, février 2000 (2000-02), pages 133-137, XP004187682 ISSN: 0378-1119 cité dans la demande
- MOORE J C ET AL: "STRATEGIES FOR THE IN VITRO EVOLUTION OF PROTEIN FUNCTION: ENZYME EVOLUTION BY RANDOM RECOMBINATION OF IMPROVED SEQUENCES" JOURNAL OF MOLECULAR BIOLOGY,GB,LONDON, vol. 272, no. 3, 26 septembre 1997 (1997-09-26), pages 336-347, XP000892797 ISSN: 0022-2836 cité dans la demande
- CRAMERI A ET AL: "DNA SHUFFLING OF A FAMILY OF GENES FROM DIVERSE SPECIES ACCELERATESDIRECTED EVOLUTION" NATURE, MACMILLAN JOURNALS LTD. LONDON, GB, vol. 391, 15 janvier 1998 (1998-01-15), pages 288-291, XP000775869 ISSN: 0028-0836
- STEMMER W P C: "Rapid evolution of a protein in vitro by DNA shuffling" NATURE, MACMILLAN JOURNALS LTD. LONDON, GB, vol. 370, 4 août 1994 (1994-08-04), pages 389-391, XP002082182 ISSN: 0028-0836
- TRUAN G ET AL: "ENHANCED IN VIVO MONOOXYGENASE ACTIVITIES OF MAMMALIAN P450S IN ENGINEERED YEAST CELLS PRODUCING HIGH LEVELS OF NADPH -450 REDUCTASE AND HUMAN CYTOCHROME B5" GENE,NL,ELSEVIER BIOMEDICAL PRESS. AMSTERDAM, vol. 125, no. 1, 30 mars 1993 (1993-03-30), pages 49-55, XP002005488 ISSN: 0378-1119 cité dans la demande
- K.H. ROUX: "USING MISMATCHED PRIMER-TEMPLATE PAIRS IN TOUCHDOWN PCR" BIOTECHNIQUES,US,EATON PUBLISHING, NATICK, vol. 16, no. 5, 1994, pages 812-814, XP000619211 ISSN: 0736-6205
- AB¹CASSIS V. ETAL.: NUCLEIC ACIDS RESEARCH, vol. 28, no. 20, 2000, pages 1-10, XP002179392

### LISTE DE SÉQUENCES

<110> Aventis Pharma SA
   Centre national de la Recherche Scientifique (CNRS).
<120> BANQUES COMBINATOIRES AMÉLIORÉES PAR RECOMBINAISON DANS LA LEVURE ET PROCÉDÉ D'ANALYSE
<130> D18972
<150> FR 00 07555
   <151> 2000-06-14
<160> 8
<170> PatentIn Ver. 2.1
<210> 1
   <211> 24
   <212> ADN
   <213> Levure
<400> 1
   cgtgtatata gcgtggatgg ccag 24
<210> 2
   <211> 16
   <212> ADN
   <213> Levure
<400> 2
   gcaccaccac cagtag 16
<210> 3
   <211> 24
   <212> ADN
   <213> Homo sapiens
<400> 3
   gcattgtccc agtctgttcc cttc 24
<210> 4
   <211> 31
   <212> ADN
   <213> Homo sapiens
<400> 4
   ccggcgctat gaccacaacc accaagaact g 31
<210> 5
   <211> 24
   <212> ADN
   <213> Homo sapiens
<400> 5
   agactgcctc ctccgggaac cccc 24
<210> 6
   <211> 22
   <212> ADN
   <213> Homo sapiens
<400> 6
   gctggatgag aacgccaatg te 22
<210> 7
   <211> 21
   <212> ADN
   <213> Homo sapiens
<400> 7
   cggggaagtc ctggcaagtg g 21
<210> 8
   <211> 24
   <212> ADN
   <213> Homo sapiens
<400> 8
   cacttccaaa tgcagctgcg ctct 24

## Description

La présente invention concerne un procédé de fabrication de banques combinatoires d'expression fonctionnelle à partir d'une banque combinatoire d'acides nucléiques appartenant à une même famille génique, comprenant une étape de clonage par recombinaison dans la levure. L'invention concerne aussi un procédé de production de protéines mosaïques fonctionnelles, et d'analyse d'une banque combinatoire d'expression fonctionnelle, par détermination d'une empreinte séquentielle pour chacune des protéines mosaïques de la banque.

La diversité des fonctions des protéines peut être vue comme le résultat de l'évolution des gènes par des événements de mutation, recombinaison et sélection (1, 2). Différentes techniques ont été développées pour tenter de reproduire, à l'échelle du laboratoire, les différentes étapes des processus d'évolution naturelle. Les approches classiques d'évolution moléculaire utilisent des étapes de mutation aléatoire et recombinaison par amplification en chaîne par polymérase (PCR) (2-5). L'évolution moléculaire est une approche qui a été utilisée avec succès en biotechnologie pour la modification de fonctions protéiques (5-12) et pour permettre une meilleure compréhension des mécanismes de reconnaissance de substrats (13). L'évolution moléculaire constitue une approche efficace pour la compréhension du rôle des zones de séquences pour la fonction protéique quand lesdites séquences ne sont pas comprises dans des zones hautement conservées, quand la structure tridimensionnelle n'est pas connue ou quand aucune information issue de techniques de modélisation n'est disponible (29).

Une méthode pour générer des protéines chimériques dérivant du cytochrome P_{450,} par recombinaison *in vivo* chez la levure, a précédemment permis d'obtenir des chimères bipartites présentant une activité monooxygénase augmentée (Bellamine et al., 1994). Dans une autre étude visant à faire évoluer une séquence nucléotidique, en fonction d'une caractéristique déterminée, la recombinaison *in vivo* a également été utilisée (EP 0934999). Cette fois, l'ADN est fragmenté, préalablement à l'étape de recombinaison, pour permettre un réarrangement entre séquences homéologues.

Afin de procéder aux expériences d'évolution moléculaire ou mélange recombinatoire d'ADN (DNA-shuffling), on part d'une banque de gènes qui peut être générée par mutagenèse d'une séquence unique (14) ou qui peut être constituée d'un groupe appartenant à une même famille ou sous-famille de gènes (15). La technique dite du mélange recombinatoire de famille (family-shuffling) a été décrite comme un moyen d'accélérer les processus d'évolution (16), qui permet l'émergence d'activités ou de propriétés inespérées dans les nouvelles protéines générées (14). Cette technique a ainsi permis la création d'enzymes présentant une association de propriétés parentales d'intérêts (17, 18), ayant une stabilité thermique accrue (14) ou présentant de nouvelles spécificités de substrat (19).

Toutefois, même si le mélange recombinatoire de gènes d'une même famille (family-shuffling) permet d'obtenir des améliorations imitant *in vitro* les processus d'évolution, la construction de banques aléatoires de structures mosaïques dépourvues de biais envers le réassemblage d'une majorité de structures parentales est toujours un point critique.

Les difficultés pour obtenir une banque homogène par «family-shuffling» augmentent fortement lorsque les similarités entre les séquences de départ utilisées diminuent (30, 31). Ainsi, un relativement petit nombre (de l'ordre de 10 %) de chimères a été fréquemment décrit (Kikuchi décrit 1% de structures chimériques pour 2 gènes ayant 84% d'identité au niveau protéique en utilisant les techniques classiques de DNA-shuffling (32)).

Différentes techniques ont été développées pour diminuer le taux de structures parentales dont l'utilisation d'ADN simple brin comme point de départ pour le mélange recombinatoire (donnant 14% de structures chimériques pour 2 gènes ayant 84% d'identité au niveau protéique (33) ou des fragmentations enzymatiques limitées (32,34) donnant, elles, des taux de chimères beaucoup plus importants. Toutefois, cette dernière méthode présente l'inconvénient que les fragments générés de façon enzymatique ne sont pas des fragments aléatoires, ce qui induit une limitation dans le nombre de nouvelles structures géniques qui peuvent ainsi être produites.

D'autres groupes ont utilisé la recombinaison *in vivo* dans des systèmes procaryotes pour obtenir des chimères (30, 35, 36). Ces méthodes présentent toutefois l'inconvénient que l'expression fonctionnelle de protéines dans E. *coli* n'est pas toujours la plus adaptée lorsqu'il s'agit de protéines eucaryotes, en particulier les complexes multiprotéiques, les protéines membranaires, ou toute protéine nécessitant la machinerie cellulaire eucaryote pour son activité. En particulier, certaines protéines eucaryotes présentent des modifications post-traductionnelles (glycosylation...) qui ne peuvent pas être effectuées dans les hôtes procaryotes.

Il est donc un objet de la présente invention que de fournir un procédé de construction de banques combinatoires d'expression fonctionnelle à partir d'acides nucléiques appartenant à une même famille génique, qui permet d'obtenir des banques présentant la complexité nécessaire, c'est-à-dire présentant une grande partie des structures chimériques possibles, et avec un taux de présence de structures parentales relativement faible. Par ailleurs, le procédé de la présente invention permet d'obtenir de banques qui permettent une meilleure expression de protéines eucaryotes.

La présente invention divulgue également un procédé d'analyse des séquences géniques d'une banque combinatoire, en particulier obtenue par le procédé selon l'invention, qui permet d'associer une « empreinte » à chaque variant de séquence présent dans ladite banque. Ce procédé d'analyse permet, en combinaison avec un procédé d'analyse des fonctions et/ou activités des protéines de ladite banque, de pouvoir relier lesdites structures séquentielles et lesdites structures fonctionnelles. Ainsi, la combinaison de ces deux procédés peut être utilisée pour « piloter » le mélange d'informations génétiques, afin d'obtenir des protéines d'intérêt de façon dirigée, plus contrôlée, plus rapidement et à un moindre coût.

Ainsi, la présente invention concerne un procédé de construction d'une banque combinatoire d'expression fonctionnelle à partir d'une banque d'acides nucléiques appartenant à une même famille génique, caractérisé en ce qu'il comporte les étapes consistant à :
a. introduire ladite banque d'acides nucléiques dans une levure, simultanément avec un vecteur d'expression,
b. obtenir ladite banque d'expression fonctionnelle par recombinaison de ladite banque combinatoire d'acides nucléiques avec ledit vecteur d'expression dans ladite levure.

Une banque combinatoire d'expression fonctionnelle obtenue par un tel procédé selon l'invention est également un objet de l'invention.

De préférence, le vecteur d'expression avec lequel est effectué la recombinaison dans la levure est linéarisé au site de clonage normal de l'ADNc et possède des séquences promotrices et terminatrices de transcription, la recombinaison s'effectuant au niveau desdites séquences.

Les fragments d'acides nucléiques appartenant à la banque introduite dans la levure à l'étape a. peuvent être fragmentés ou non. Lorsque ces fragments sont fragmentés, ceci permet d'augmenter l'efficacité de recombinaison *in vivo,* ce qui augmente la diversité de la banque, dans la mesure où un événement de recombinaison est obligatoire avant le clonage dans le vecteur d'expression. ces points seront discutés plus loin.

Les événements de recombinaison s'effectuant dans la levure peuvent être de recombinaison homologue (entre séquences identiques) ou homéologue (entre séquences présentant un niveau d'identité suffisant).

Le procédé selon l'invention est également très intéressant en ce qu'il ne nécessite pas d'étape de passage dans un procaryote, pour l'obtention de la banque combinatoire.

Ainsi, le procédé selon la présente invention permet l'obtention d'une banque combinatoire d'expression directement dans un hôte eucaryote, ce qui présente un avantage certain pour l'expression de protéines eucaryotes, en particulier les protéines membranaires, ou appartenant à des complexes multiprotéiques.

Le procédé selon la présente invention concerne donc une méthode de production de banques combinatoires améliorées par recombinaison dans une levure (CLERY, pour Combinational Library Enhanced by Recombination in Yeast).

La levure (qui peut être modifiée au niveau génomique) est également avantageusement utilisée comme outil d'expression (39) des gènes chimériques, permettant d'améliorer l'expression fonctionnelle des nouvelles protéines eucaryotes obtenues par cette méthode (en particulier les complexes multiprotéiques ou les protéines membranaires). Par ailleurs, la modification génomique de la souche de levure utilisée peut permettre de recréer l'environnement naturel de fonctionnement (et donc l'optimisation des possibilités de criblage), par production d'autres protéines eucaryotes essentielles pour l'activité des nouvelles protéines créées, en particulier dans les cas de complexes multiprotéiques.

Le procédé selon l'invention permet l'obtention finale d'une banque combinatoire d'expression fonctionnelle du fait de deux étapes différentes :
- le clonage de la banque d'acides nucléiques dans le vecteur d'expression introduit simultanément dans la levure, par recombinaison homologue *in vivo,* permet d'obtenir une banque d'expression fonctionnelle ;
- la recombinaison homologue ou homéologue (entre les séquences semblables mais non identiques), pouvant se produire *in vivo* dans la levure, entre les différents acides nucléiques de la banque combinatoire introduite dans ladite levure, permet d'augmenter la complexité et la diversité de la banque combinatoire d'expression fonctionnelle obtenue.

Ainsi, lorsque les fragments d'acides nucléiques de la banque combinatoire introduite dans ladite levure sont fragmentés et ne possèdent pas les deux extrémités recombinogènes permettant le clonage dans le vecteur d'expression, il est essentiel qu'un événement de recombinaison entre deux fragments adéquats ait lieu préalablement audit clonage.

De même dans un cas particulier de mise en oeuvre du procédé selon l'invention, on observe la réalisation d'au moins un événement de recombinaison homéologue dans la banque obtenue, notamment du fait de l'appartenance à une même famille génique des acides nucléiques de la banque initialement introduite dans la levure.

Par « acides nucléiques appartenant à une même famille génique », on entend, au sens de l'invention, des acides nucléiques possédant au minimum 35 % d'identité, de manière préférée 40 %, de manière plus préférée 50 %, ou encore 70 %. Ces acides nucléiques seront dit appartenir à la même famille génique s'ils présentent les pourcentages d'identités ci-dessus, et peuvent coder pour des protéines présentant des activités et/ou fonctions différentes. Ces acides aminés peuvent coder pour des protéines trouvées naturellement, ou être des acides nucléiques « artificiels », c'est-à-dire codant pour des protéines qui ne sont pas trouvées dans la nature. En particulier de tels acides nucléiques « artificiels » englobent des protéines de fusion, ou des protéines déjà obtenues par des méthodes de mélange recombinatoire d'ADN.

Par « pourcentage d'identité » entre deux séquences d'acides nucléiques ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (49), au moyen de l'algorithme d'homologie locale de Neddleman et Wunsch (50), au moyen de la méthode de recherche de similarité de Pearson et Lipman (51), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62. On peut également utiliser les matrices PAM ou PAM250.

La présente invention permet ainsi d'obtenir, avec un rendement élevé, des banques recombinatoires à partir d'acides nucléiques présentant une identité très inférieure à l'identité actuellement requise dans l'état de la technique (généralement supérieure à 70 %).

La banque d'acides nucléiques introduite dans la levure à l'étape a. du procédé selon l'invention est, de préférence, elle même une banque combinatoire d'acide nucléiques.

Cette banque d'acides nucléiques est, de préférence, un mélange de produits de PCR obtenu par amplification d'une banque combinatoire de phases ouvertes de lecture, en utilisant un couple d'amorces situées dans des régions flanquant lesdites phases ouvertes de lecture. Cette banque combinatoire de phases ouvertes de lecture est obtenue à partir d'ADN variants de séquences, différant par une ou plusieurs mutations, et appartenant à une même famille génique au sens de l'invention.

On utilise de préférence un seul couple d'amorce pour effectuer la réaction de PCR telle que décrite dans le paragraphe précédent, mais l'homme du métier pourrait également utiliser des couples d'amorces différentes. Il est toutefois plus pratique d'utiliser un unique couple d'amorces.

En particulier, on utilise un couple d'amorces situé dans des régions promotrices et terminatrices de la traduction chez la levure, régions permettant l'expression de phases ouvertes de lectures dans cet organisme. Ainsi, il est probable que ces régions, qui seront présentes sur tous les fragments d'ADN de la banque d'acides nucléiques introduite dans la levure, seront les séquences nucléiques impliquées dans la recombinaison avec les séquences homologues du vecteur d'expression co-introduit, qui permettront le clonage des phases ouvertes de lecture dans ledit vecteur, et la formation de la banque d'expression fonctionnelle.

Ainsi que précisé plus avant, la banque d'acides nucléiques introduite dans la levure est, de préférence, elle même une banque combinatoire d'acides nucléiques, appartenant à une même famille génique au sens de l'invention. On peut obtenir cette banque combinatoire par des méthodes classiques de fragmentation d'ADN et de réassemblage par extension par amorce (« primer extension »).

L'étape de fragmentation de l'ADN est effectuée par des méthodes connues de l'homme du métier, comme par exemple la digestion par enzymes de restriction, ou la nébulisation. On préfère toutefois fragmenter l'ADN par digestion partielle avec une DNase, de préférence la DNaseI, qui permet d'obtenir de manière plus contrôlée des fragments d'une taille désirée. Par ailleurs, ceci permet d'obtenir effectivement des fragments aléatoires, ce qui n'est pas toujours le cas avec les autres techniques de fragmentation enzymatique. Dans la pratique, et afin d'obtenir une banque combinatoire présentant une grande variété de combinaison et un grand nombre de protéines mosaïques différentes, on cherche à obtenir des fragments de taille comprise entre 15 et 700 paires de bases (pb), de préférence de 40 à 500 pb, de façon plus préférée de 100 à 300 pb.

Les fragments sont réassemblés entre eux par une technique d'extension par amorces (« primer extension »). Dans le principe, les fragments obtenus peuvent s'hybrider, et l'ajout d'une ADN polymérase permet d'obtenir une extension des fragments hybridés, et la reconstitution de gènes fonctionnels, par plusieurs cycles d'extension.

Ainsi, la présente invention a également pour objet un procédé de construction d'une banque combinatoire d'expression fonctionnelle à partir d'une banque combinatoire d'acides nucléiques appartenant à une même famille génique, comportant les étapes consistant à :
a. introduire ladite banque combinatoire d'acides nucléiques dans une levure, simultanément avec un vecteur d'expression,
b. obtenir ladite banque d'expression fonctionnelle par recombinaison de ladite banque combinatoire d'acides nucléiques avec ledit vecteur d'expression dans ladite levure,
ladite banque combinatoire d'acides nucléiques étant un mélange de produits de PCR obtenu par amplification d'une banque combinatoire de phases ouvertes de lecture, en utilisant un couple d'amorces situées dans des régions flanquant lesdites phases ouvertes de lecture, ladite banque combinatoire étant obtenue à partir d'ADN homologues ou variants de séquence différant par une ou plusieurs mutations, et ladite banque combinatoire de phases ouvertes de lecture est obtenue par réassemblage par « primer extension » de produits de fragmentation d'au moins deux phases ouvertes de lectures codant pour des protéines fonctionnelles, lesdites phases ouvertes de lectures présentant entre elles une identité de séquence supérieure à 40 %.

L'homme du métier connaît d'autres techniques permettant la recombinaison entre fragments d'ADN et leur mélange (DNA shuffling). Ainsi, une méthode alternative est la méthode d'oligo-ligature, qui peut éventuellement être mise en oeuvre avec des ligases thermostables. D'autres méthodes adéquates peuvent être choisies par l'homme du métier pour le mélange des acides nucléiques.

Afin de réaliser l'assemblage des fragments, on utilise de préférence une réaction d'amplification par polymérase (PCR). Les différentes étapes de cette réaction doivent être contrôlées, afin de pouvoir obtenir un taux important de gènes mosaïques. Ainsi, l'étape d'hybridation est une étape très importante pour assurer la possibilité d'obtenir une recombinaison entre des fragments présentant une identité de séquence relativement faible, en particulier pour les valeurs basses des gènes appartenant à une même famille génique (35 %, ou 40 %). Ainsi, la réaction de PCR mise en oeuvre de façon préférée lors de l'étape de réassemblage est caractérisée en ce que chacun de ses cycles présente au moins deux paliers d'hybridation, de préférence au moins quatre paliers, par températures décroissantes régulièrement espacées. Il est également important que l'ensemble des étapes d'hybridation ait une durée totale de plus de quatre minutes. Un mode particulier de mise en oeuvre de la réaction de PCR est tel que chaque cycle présente au moins quatre paliers d'hybridation de plus de 60 secondes, par températures décroissantes régulièrement espacées.

Les Inventeurs ont en effet montré que ces conditions de réassemblage permettent l'obtention de fragments d'une taille supérieure aux acides nucléiques de départ. En particulier, lorsque les acides nucléiques de départ sont des vecteurs d'expression portant les gènes de la même famille génique, les étapes de fragmentation et de réassemblage peuvent permettre d'obtenir des fragments d'ADN transformants dans la levure, c'est à dire portant à la fois des gènes mosaïques, et les éléments du vecteur permettant sa réplication et son maintien dans la levure. Ceci assure que la méthode de réassemblage selon le présent procédé est extrêmement efficace (voir aussi les exemples).

Afin d'obtenir une banque d'expression fonctionnelle dans la levure, le procédé selon la présente invention propose la co-introduction d'un vecteur d'expression et d'une banque d'acides nucléiques appartenant à une même famille génique, obtenue par « family shuffling » ainsi que décrit dans les paragraphes précédents.

Afin d'obtenir ladite banque d'acides nucléiques, il est intéressant de partir d'acides nucléiques appartenant à une même famille génique déjà clonés dans un vecteur d'expression de préférence, ces acides nucléiques sont tous clonés dans le même vecteur d'expression, et on utilise ledit vecteur pour la co-introduction dans la levure.

Ainsi, après l'étape de réassemblage décrite précédemment, et dans la mesure où les conditions utilisées permettent d'obtenir de longs fragments, en particulier de taille égale ou supérieure à la taille du vecteur de départ (c'est-à-dire plus longs que les acides nucléiques appartenant à la même famille génique que l'on cherche à mélanger), on effectue une réaction de PCR en utilisant un couple d'amorces situées dans les régions flanquantes des phases ouvertes de lecture. Il s'agit de préférence d'amorces situées dans le vecteur d'expression, et on les choisit en particulier dans les régions promotrice et terminatrice de transcription dudit vecteur, ainsi qu'il a été précisé précédemment.

En tant qu'ADN de départ, on peut ainsi utiliser tout vecteur contenant les acides nucléiques appartenant à la même famille génique que l'on désire recombiner. On peut choisir un vecteur multicopie dans la levure, ou un vecteur monocopie dans la levure, ou un vecteur dont le caractère multi- ou mono- copie soit inductible. On peut aussi choisir un vecteur d'expression pour une levure ou un vecteur d'expression pour une cellule eucaryote, qui soit navette pour la levure. On peut également choisir un vecteur qui contient les éléments nécessaires pour se répliquer de façon autonome chez *Escherichia coli.* On peut bien entendu prendre un vecteur qui ne possède aucune des propriétés développées ci-dessus, ou qui présente une combinaison desdites propriétés.

De préférence, on met en oeuvre le procédé selon l'invention en choisissant, comme vecteur de départ, le vecteur d'expression co-introduit dans la levure avec la banque d'acides nucléiques.

Ce vecteur d'expression possède les éléments pour se répliquer de façon autonome dans la levure, en tant que vecteur multicopie, ou vecteur monocopie, ou vecteur conditionnel. Il peut également posséder des gènes permettant sa sélection sur milieux appropriés, en particulier des gènes de résistance aux antibiotiques ou de complémentation d'auxotrophie si la levure utilisée présente cette propriété.

Le vecteur d'expression peut être un vecteur d'expression pour la levure. Dans ce cas, il possède des éléments permettant la transcription et la traduction de manière efficace dans la levure. Il peut alternativement être un vecteur d'expression dans un autre hôte, procaryote ou eucaryote, c'est-à-dire posséder les éléments (origines de réplication) lui permettant de se répliquer de façon autonome dans cet autre hôte. On choisit de préférence un vecteur qui permet l'expression dans un hôte eucaryote supérieur, en particulier une cellule de mammifère. Un tel vecteur associe, à une cassette d'expression d'eucaryote supérieur, une origine de réplication et un marqueur de sélection pour la levure.

Le vecteur comporte de préférence un promoteur, des signaux d'initiation et de terminaison de la traduction, ainsi que des régions appropriées de régulation de la transcription. Il peut éventuellement posséder des signaux particuliers spécifiant la sécrétion de la protéine traduite. Les vecteurs qui peuvent être utilisés sont bien connus de l'homme du métier.

On utilise de préférence, comme vecteur portant les acides nucléiques appartenant à une même famille génique que l'on désire fragmenter, un vecteur qui présente une taille, incluant les phases ouvertes de lecture, supérieure à 7 kilobases (kb). On peut utiliser le même vecteur pour la co-introduction dans la levure, pour l'étape de recombinaison dans la levure.

La recombinaison est effectuée dans la levure, de préférence une levure du genre *Saccharomyces,* de façon plus préférée *S. cerevisiae.* On peut toutefois utiliser d'autres types de levures, parmi lesquels *Candida, Yarrovia, Kluyveromyces, Schizosaccharomyces, Torulopsis, Pichia, Hansenula.* L'homme du métier choisira la levure appropriée en fonction de ses compétences et connaissances et de l'objectif recherché. Cette levure peut être modifiée au niveau génomique pour exprimer des protéines exogènes, permettant de complémenter les protéines mosaïques que l'on cherche à générer.

Le procédé selon la présente invention possède plusieurs avantages, qui seront en particulier visibles à la lumières des exemples. Toutefois, on peut en résumer certains :
- le procédé ne nécessite pas de passage dans un hôte procaryote pour l'obtention de la banque, ce qui simplifie les manipulations à effectuer ;
- le procédé selon l'invention permet, en une étape, de procéder au clonage dans le vecteur d'expression de la banque d'acides nucléiques introduite dans la levure, et d'augmenter la diversité par recombinaison homologue ou homéologue entre les différents acides nucléiques de la banque combinatoire introduite dans la levure ;
- lorsque le vecteur d'expression est multicopie, on obtient un mélange de produits dans la levure, consistant en plusieurs copies dudit vecteur, possédant chacune un gène mosaïque différent. Chaque clone de levure obtenu contient donc individuellement une banque de gènes mosaïques, et ceci permet de tester les activités des différentes protéines de façon plus rapide et efficace ;
- lorsque le vecteur d'expression peut également se répliquer chez *E. coli,* on peut alors effectuer la ségrégation des différents plasmides en préparant l'ADN plasmidique d'au moins un clone de levure obtenu, en transformant *E. coli* avec ledit ADN plasmidique extrait, et en sélectionnant les clones transformés sur milieu approprié pour obtenir une discrimination des éléments de la banque combinatoire d'expression fonctionnelle ;

Ainsi, l'homme du métier désirant améliorer les propriétés fonctionnelles d'une protéine pourra préparer, par le procédé selon l'invention, une banque combinatoire d'expression fonctionnelle dans la levure à partir d'acides nucléiques d'intérêt appartenant à une même famille génique. Il pourra ensuite tester les clones de levures pour sélectionner ceux pour lesquels la propriété recherchée est apparente, et obtenir les séquences réellement intéressantes en effectuant la discrimination par passage dans un hôte procaryote.

Le procédé selon l'invention permet ainsi de produire des protéines fonctionnelles mosaïques actives, elles-mêmes objets de l'invention. Ainsi, un procédé de production de protéines fonctionnelles mosaïques actives, caractérisé en ce que l'on construit une banque combinatoire d'expression fonctionnelle par un procédé selon l'invention, que l'on exprime les protéines mosaïques, et que l'on sélectionne les protéines fonctionnelles mosaïques actives par l'étude de leur activité, est également un objet de l'invention.

De préférence, les protéines mosaïques que l'on cherche à générer sont des enzymes possédant des activités améliorées (thermostabilité, fonction nouvelle, modification de fonction, augmentation d'activité, modification de la spécificité de substrat, modification de l'activité dans un environnement précis tel un solvant, un pH...). L'utilisation du procédé selon l'invention pour générer de nouvelles enzymes présente beaucoup d'avantages, puisque les activités des nouvelles protéines générées peuvent alors souvent être testées directement dans la levure. On utilise alors de préférence comme acides nucléiques de départ, des acides nucléiques appartenant à une même famille génique, qui codent pour des enzymes. Les protéines mosaïques actives obtenues sont alors dites dérivées d'enzymes.

Les exemples de la présente invention montrent l'application du procédé à la génération de nouvelles protéines dérivées de cytochromes P450s. Les cytochromes P450s (P450s) peuvent reconnaître une large variété de substrats et catalyser un nombre encore plus grand de réactions. Ces enzymes ont été mis en évidence dans pratiquement tous les organismes vivants (20). Chez les mammifères, les P450s sont impliqués dans la formation d'hormones stéroïdes mais ont également un rôle prédominant dans le métabolisme des médicaments et des polluants qui peuvent parfois amener à des processus de toxicité et de carcinogenèses chimiques (20-22). Les P450s 1A1 et 1A2 humains ont de l'ordre de 70 % d'identité de séquence et possèdent certaines spécificités de substrats différentes. Ils sont parmi les P450s les plus actifs dans le métabolisme des carcinogènes chimiques (23) et sont impliqués, chez l'homme, dans le cancer du poumon pour le *CYP1A1* (24-26), dans l'activation de promutagènes contenus dans la nourriture (27) ou dans les cancers du foie induits par l'aflatoxine B1 pour le *CYP1A2.* L'ensemble des propriétés des P450s de mammifères en fait en effet d'excellents candidats pour l'application de ces techniques d'évolution moléculaire (28).

Un cas particulier de la présente invention concerne donc le procédé selon la présente invention, caractérisé de plus en ce que le vecteur d'expression eucaryote utilisé pour le mélange recombinatoire contient une phase ouverte de lecture codant pour un enzyme membranaire eucaryote. De préférence, ledit enzyme eucaryote est choisi dans le groupe constitué des cytochromes P450 eucaryotes, enzymes de conjugaison eucaryotes (de phase II), membres de la famille des transporteurs ABC eucaryotes.

Dans ce cas, il peut être intéressant d'utiliser une souche de levure qui présente une modification génétique permettant la surexpression d'au moins une protéine choisie dans le groupe constitué d'une P450 réductase endogène ou exogène, une adrénodoxine, une adrénodoxine réductase, un cytochrome b5 hétérologue, une enzyme de phase II (en particulier une époxide hydrolase). De telles souches sont décrites dans le brevet EP 595 948. Ces souches permettent en particulier permet de recréer l'environnement naturel de fonctionnement des P450s eucaryotes (40,41).

L'utilisation de souches de levures génétiquement modifiées permet en outre de recréer des complexes protéiques, avec plusieurs éléments fixes (exprimés de façon constitutive par la levure), et un élément variable (le produit des gènes mosaïques obtenus par le procédé selon l'invention).

Le procédé selon la présente invention peut aussi être appliqué à d'autres protéines. Par exemple, il peut être intéressant de générer des récepteurs, ce qui permet de déterminer les séquences impliquées dans la reconnaissance et l'association du ligand, ou des protéines chimères basées sur les protéines cibles des antibiotiques, qui permettent de déterminer les degrés de résistance en fonction des mutations.

De façon habituelle, il est nécessaire d'effectuer de nombreux cycles de « DNA-shuffling » avant d'obtenir une protéine présentant les caractéristiques et/ou propriétés désirées. Dans le cas présent, après sélection des clones de levure exprimant des protéines ayant une activité proche de l'activité recherchée, il est possible d'effectuer une simple réaction de PCR directement sur lesdits clones, en utilisant des amorces appropriées flanquant les phases ouvertes de lecture, et de procéder à un nouveau mélange recombinatoire en répétant les étapes du procédé selon l'invention.

Il est toutefois souhaitable de pouvoir améliorer la rapidité d'obtention des propriétés désirées, en effectuant une relation entre les structures séquentielles des protéines mosaïques obtenues, et les structures fonctionnelles desdites protéines. Ceci permet alors de relier facilement les séquences d'ADN du gène, ou les liaisons entre les séquences, à une fonction enzymatique ou autre (attachement d'un substrat, thermophilie...).

La présente invention concerne donc également un procédé d'analyse d'une banque combinatoire d'expression fonctionnelle, caractérisé en ce qu'il comprend les étapes suivantes :
a. transformation d'une souche *d'Escherichia coli* avec l'ADN plasmidique extrait de la souche de levure ou d'un pool de levures,
b. hybridation de l'ADN plasmidique contenu dans chacun des clones individuels *d'Escherichia coli* obtenus à l'issu de l'étape

a. avec une ou plusieurs sondes spécifique(s) d'une séquence parentale.

Ce procédé, amélioré des étapes qui seront décrites ultérieurement, peut être utilisé sur toute banque combinatoire, à partir du moment où les différents acides nucléiques formant la banque ont été discriminés.

L'hybridation s'effectue sur un macro- ou un micro-réseau d'ADN, ledit réseau étant constitué soit de l'ADN plasmidique contenu dans chacun des clones individuels *d'Escherichia coli* obtenus à l'issu de l'étape a., ou d'un produit de PCR de celui-ci, soit desdites sondes spécifiques, attaché(es) sur un support solide, chacun des acides nucléiques étant repéré par sa position dans ledit réseau.

Dans le premier cas, on fixe l'ADN plasmidique contenu dans chacun des clones individuels *d'Escherichia coli* obtenus à l'issu de l'étape a., ou un produit de PCR de celui-ci sur un support solide (verre, silicium, membrane appropriée (Nylon, nitrocellulose)...). Les méthodes de fixation de l'ADN sont connues de l'homme du métier, et l'ADN peut être fixé de manière plus ou moins solide sur le support utilisé. Il n'est pas toujours nécessaire d'extraire l'ADN plasmidique des clones d'*E*. *coli* obtenus, ceux-ci pouvant être directement lysés sur le support solide utilisé, ou la PCR permettant l'amplification des fragments correspondant aux gènes mosaïques pouvant être effectuée directement sur les clones bactériens sans extraction d'ADN préalable.

Dans le second cas, on fixe les sondes sur le support solide. Il existe plusieurs méthodes pour préparer un support portant des sondes. On peut synthétiser les sondes puis les fixer sur le support (l'adressage pouvant se faire mécaniquement, électroniquement, par jet d'encre...) ou synthétiser les sondes directement sur le support (par adressage photochimique ou par jet d'encre, par exemple). L'homme du métier choisira la méthode la plus appropriée pour le résultat recherché.

En fonction du nombre de sondes utilisées, on obtient une empreinte d'hybridation plus ou moins fine, pour chacun des clones testés. Plus le nombre de sondes est élevé, plus l'empreinte obtenue sera fine. On peut choisir des sondes qui sont situées de façon homogène sur toute la longueur du gène. Alternativement, il peut être profitable d'utiliser des sondes qui sont ciblées dans un ensemble de zones de séquences dont on sait qu'elles codent pour des régions importantes pour la fonction et/ou l'activité de la protéine. Ainsi, on peut obtenir une empreinte séquentielle ciblée.

Par ailleurs, les conditions d'hybridation des sondes varient en fonction du degré de spécificité desdites sondes pour chaque structure parentale. Ainsi, lorsque deux structures parentales diffèrent d'une simple base sur le fragment correspondant à la sonde, il est nécessaire d'appliquer des conditions de stringence plus élevées que si les structures parentales sont très différentes. L'homme du métier sait déterminer les meilleures conditions d'hybridation, en particulier en suivant l'enseignement de Sambrook *et al.* Il est également important de noter que certaines gènes mosaïques peuvent présenter une intensité d'hybridation avec une sonde donnée moindre que d'autres gènes. En effet, l'efficacité du transfert de l'ADN sur le support solide peut s'être effectuée de manière plus ou moins efficace, ou bien la région du gène sur laquelle la sonde doit s'hybrider est elle-même mosaïque et constituée de fragments provenant de gènes « parents » différents.

On peut alors procéder à une analyse statistique des intensités d'hybridation, à l'aide d'un programme informatique approprié. Le programme convertit d'abord les signaux d'hybridations en données d'un type parental par un système de masques avec une fonction booléenne XOR avant l'analyse statistique proprement dite.

L'analyse de la banque combinatoire peut s'effectuer de la façon suivante :
- Un code est attribué à chaque séquence nucléique générée, en fonction de la capacité des sondes utilisées à hybrider ladite séquence. Il peut être avantageux d'utiliser un codage binaire (0 si l'endroit sondé correspond à un certain type parental, 1 s'il correspond à l'autre type parental), mais d'autres types de codages peuvent aussi être utilisés. Ainsi, chaque séquence générée dans la banque possède une « signature » individuelle. Dans le cas où 6 sondes sont utilisées et l'on utilise un codage binaire, 2⁶ possibilités sont envisagées (de 000000 à 111111)
- La fréquence de chacune des signatures ainsi obtenues est alors comparée avec la fréquence attendue si le mélange recombinatoire d'ADN s'effectuait complètement au hasard (dans le cas de 6 sondes, la fréquence théorique de chaque pattern est alors de 1/2⁶). Cette analyse permet de définir un « parent préférentiel » pour chacune des positions sondées (certaines corrections doivent parfois être apportées, en particulier lorsque les proportions d'acides nucléiques parentaux de départ ne sont pas égales).
- L'étude des signatures permet également de préciser les relations qui peuvent exister au sein d'une même mosaïque, en particulier les associations entre types parentaux qui peuvent être trouvées entre chaque segment. Par exemple, il est important de pouvoir aisément déterminer la nécessité d'une corrélation entre deux segments nucléiques non nécessairement adjacents pour l'obtention d'une fonction biologique.
- L'analyse peut également être affinée afin d'obtenir des résultats qui peuvent fournir plusieurs renseignements. Les exemples illustrent une telle étape en divulguant une méthode où chaque signature de la banque est convertie en un nombre décimal, et où une courbe, portant en abscisse ledit nombre décimal et la fréquence cumulée en ordonnée, est dessinée. L'analyse de ladite courbe, et sa modélisation par simulation permet également d'obtenir des renseignements intéressant sur la probabilité d'obtenir un certain type de structure parentale à un endroit donné, et les corrélations existant entre différents fragments.

Les analyses statistiques ainsi décrites sont facilitées grâce à l'utilisation d'outils informatiques, dont le développement ne pose pas de problèmes à l'homme du métier.

Les simulations de corrélations entre divers segments peuvent être effectuées en générant des grilles plus ou moins aléatoires, selon que les corrélations désirées. Par exemple, une grille peut être générée pour laquelle un segment a une probabilité supérieure à 50 % d'être du même type parental que le segment voisin. Le nombre de grilles qui peuvent ainsi être générées est extrêmement important, et peut permettre ainsi de définir une approximation des résultats observés.

Lorsque l'on observe des corrélations entre différents segments, il est probable que l'application d'une sélection fonctionnelle sur la population de clones (qui réduit ainsi la population de séquences passant le crible) mènera à une augmentation du nombre de corrélations, et à une évolution (convergence) des résultats statistiques obtenus. On devrait donc obtenir l'apparition d'un pattern caractéristique de la sélection appliquée, ce qui donne une signature séquentielle dépendant de la sélection fonctionnelle appliquée sur le système.

En résumé, la présente invention concerne également un procédé d'analyse des empreintes d'hybridation pouvant être obtenues par le procédé d'analyse de la banque combinatoire décrit ci-dessus, caractérisé en ce qu'il comprend les étapes consistant à :
a. calculer la fréquence d'apparition de chacune des combinaisons possibles,
b. définir une signature de la répartition statistique des combinaisons, par un traitement mathématique et statistique adéquat.

Ainsi, la présente invention fournit un moyen de produire de façon très efficace des banques combinatoires d'expression fonctionnelle à partir d'acides nucléiques appartenant à une même famille génique au sens de l'invention, qui peuvent posséder un degré d'identité relativement faible.

Par ailleurs, la présente invention présente l'avantage de pouvoir effectuer le test des activités des protéines mosaïques produites, directement sur les clones de levures obtenus, sans étape préalable de purification.

La présente invention fournit également un procédé d'analyse de banques combinatoire, basé sur une hybridation et une analyse statistique des empreintes d'hybridations obtenues.

La présente invention fournit donc des outils qui peuvent être mis en oeuvre pour la détermination des liens qui peuvent exister entre les structures séquentielles et les structures fonctionnelles des protéines. Ainsi, la présente invention concerne également un procédé de détermination de liens entre signatures séquentielles et signatures fonctionnelles d'une protéine, caractérisé en ce qu'il comprend les étapes consistant à
a. préparer une banque combinatoire d'expression fonctionnelle par un procédé selon l'invention,
b. produire les protéines fonctionnelles mosaïques actives,
c. analyser les différences fonctionnelles et/ou d'activité entre lesdites protéines mosaïques,
d. analyser les acides nucléiques correspondant auxdites protéines mosaïques par un procédé d'analyse par hybridation selon l'invention, suivi de façon optionnelle par une analyse statistique par un procédé selon l'invention,
e. relier les différences de structure séquentielle observées dans l'étape d. avec les différences fonctionnelles et/ou d'activité observées dans l'étape c.

La mise en oeuvre de ce procédé, pour identifier les zones de séquences importantes ou les liens entre zones de séquences reliés à une fonction d'intérêt permet de prédire les structures ayant ladite fonction, par déduction de la structure recherchée, en fonction de la relation structure-fonction obtenue par le procédé décrit ci-dessus.

Ainsi, il devient possible d'obtenir des protéines qui possèdent des propriétés améliorées, telles que décrites précédemment, ou des protéines qui reconnaissent un grand nombre de substrat (enzymes « génériques »), en pilotant les mélanges d'informations génétiques, afin d'obtenir les protéines d'intérêt plus rapidement, et de façon plus efficace.

Les différents procédés décrits dans l'état de la technique permettaient d'obtenir les protéines d'intérêt par une répétition des mélanges recombinatoires d'ADN, en soumettant les protéines obtenues à des cribles de plus en plus fins. La présente invention, permettant de relier les structures et les fonctions des protéines mosaïques obtenues, permet de procéder à des nouveaux mélanges recombinatoires en n'utilisant, comme acides nucléiques de départ, que les acides nucléiques ayant été identifiés comme portant les structures ou organisations de structures d'intérêt.

Ainsi, la présente invention concerne un procédé d'obtention d'une protéine possédant des propriétés améliorées, caractérisé en ce qu'il comprend les étapes consistant à :
a. construire une banque combinatoire d'expression fonctionnelle par un procédé selon l'invention,
b. analyser ladite banque combinatoire d'expression fonctionnelle,
c. analyser les empreintes d'hybridation obtenues dans l'étape b. par un procédé selon l'invention,
d. déterminer les liens entre les entre structures séquentielles et les structures fonctionnelles des protéines par comparaison desdites empreintes d'hybridation avec les propriétés des protéines mosaïques correspondantes,
e. prédire les structures d'intérêt ou les organisations de structures dans les protéines mosaïques,
f. répéter les étapes a. à e., en utilisant, comme acides nucléiques de départ pour la génération de la banque combinatoire d'expression fonctionnelle, les acides nucléiques portant les structures d'intérêt ou les organisations de structures identifiées dans l'étape e., un nombre suffisant de fois pour obtenir la protéine possédant des propriétés améliorées recherchées.

L'étape f. consiste à répéter les étapes précédentes jusqu'à ce qu'une protéine présentant les propriétés désirées ait pu être identifiée. La présente invention, devrait permettre de diminuer le nombre de cycles de fabrication de banque combinatoire - analyse des protéines par rapport aux méthodes de l'art antérieur.

Les protéines obtenues par le procédé décrit sont également un objet de l'invention.

L'invention se rapporte également à un procédé de détermination d'une structure d'une protéine importante en réponse à une pression de sélection à partir d'une banque combinatoire d'expression fonctionnelle obtenue par un procédé selon l'invention, pour les éléments de laquelle une signature a été obtenue, comprenant les étapes de :
- normaliser ladite banque, par l'homogénéisation des signatures, par exemple par tri à l'aide d'un appareil robotique approprié. Cette étape permet d'assurer que chaque empreinte se retrouve avec la même probabilité dans la banque normalisée.
- appliquer une pression de sélection,
- analyser la banque d'expression résultante en mettant en oeuvre les procédés d'analyse de signature de séquence selon l'invention,
- étudier les changements de signatures de séquence induits par la pression de sélection sur la banque normalisée initiale et en déduire les structures sélectionnées ou contre sélectionnées en réponse à la pression de sélection.

Il est à noter que le fait de normaliser la banque avant d'appliquer la pression de sélection permet en fait de cribler une diversité plus importante en criblant le même nombre de clones que si l'on ne normalise pas. En effet, on peut remarquer que certaines structures (telles qu'analysées par les empreintes) sont présentes à des probabilités supérieures à ce qui serait attendu en cas de mélange aléatoire. La normalisation permet donc de diminuer l'influence de ce problème.

Les exemples qui suivent sont limités à la génération de nouveaux cytochromes P450s, pour illustrer l'invention. Toutefois, ils ne doivent pas être considérés comme limitant l'invention, et en particulier le type de protéines et d'acides nucléiques pouvant être utilisés dans les procédés décrits dans la présente l'invention. L'homme du métier peut ainsi aisément mettre en oeuvre les procédés de l'invention, en substituant d'autres gènes aux gènes de cytochromes P450 décrits dans les exemples.

### DESCRIPTION DES FIGURES

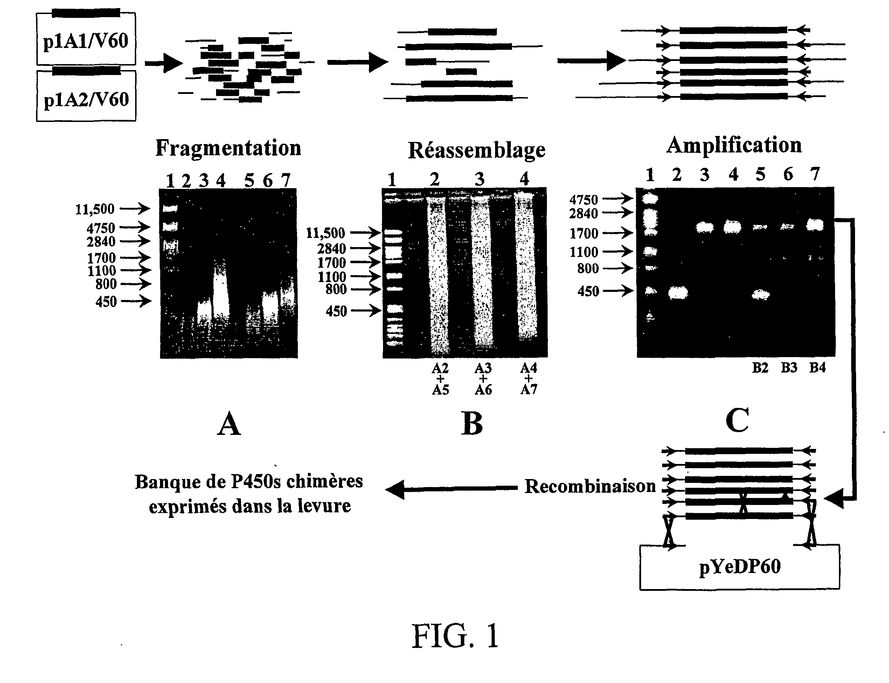

**Figure 1** : principe de la construction des banques. A: ligne 1, marqueur ADN (ADN de λ digéré par *Pst* I) ; les lignes 2, 3, 4 et 5, 6, 7 correspondent respectivement aux plasmides p1A1/V60 et p1A2/V60 digérés à la DNAse I. Les lignes 2 et 5 correspondent à la fragmentation avec 0.0112 unités, les lignes 3 et 6 avec 0.0056 unités et les lignes 4 et 7 à 0.0028 unités de DNase 1 par µg d'ADN. B: réaction de réassemblage. Ligne 1, marqueur ADN ; les lignes 2, 3 et 4 correspondent aux réactions de réassemblage entre fragments de p1A1/V60 et p1A2/V60 en mélangeant respectivement les réactions des lignes 2 et 5,3 et 6, 4 et 7. C: réaction d'amplification. Ligne 1, marqueur ADN ; les lignes 2, 3 et 4 correspondent respectivement à l'amplification avec les plasmides PYeDP60, p1A1/V60 et p1A2/V60; les lignes 5, 6 et 7 correspondent à l'amplification avec l'ADN préalablement réassemblé utilisé comme matrice (lignes B2, B3 et B4). La bande présentée en ligne 6, panel C a été purifiée et utilisée comme telle pour cotransformer *S*. *cerevisiae* avec du plasmide pYeDP60 préalablement linéarisé. On observe l'existence d'événements de recombinaison entre les différents acides nucléiques de la banque introduite dans la levure.

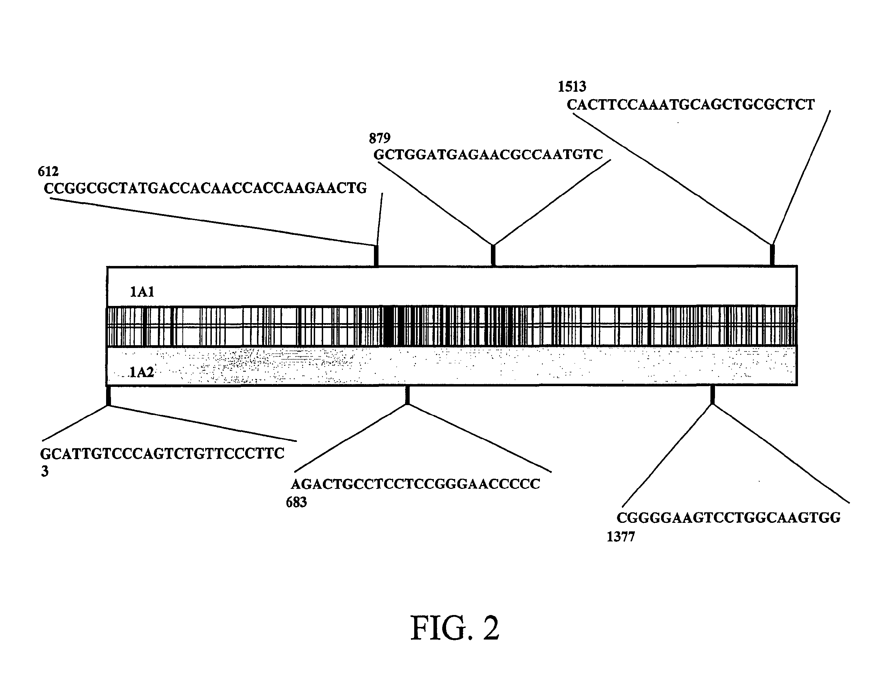

Figure 2 : positions respectives et séquences des six sondes utilisées pour réaliser les matrices de caractérisations de la banque. Les nombres sur le haut ou le bas correspondent à la position 5' d'alignement de chaque sonde sur les séquences. Les sondes sur le haut et le bas hybrident respectivement les séquences du P450 1A1 ou du P450 1A2. Les barres verticales dans le rectangle central représentent toutes les positions de misappariement entre la séquence du P450 1A1 et du P450 1A2.

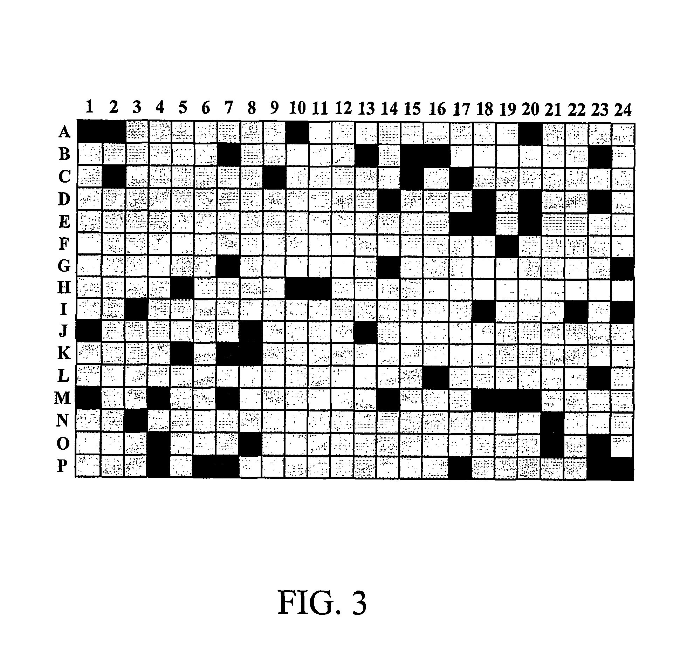

Figure 3 : Les résultats d'hybridation ont été traités dans Microsoft Excel en générant une grille de 384 points avec le code couleur suivant : les carrés foncés représentent des structures assimilées à des structures de types parentales (1A1 ou 1A2) pour les zones de séquences correspondants aux six sondes et les carrés clairs représentent des structures mosaïques.

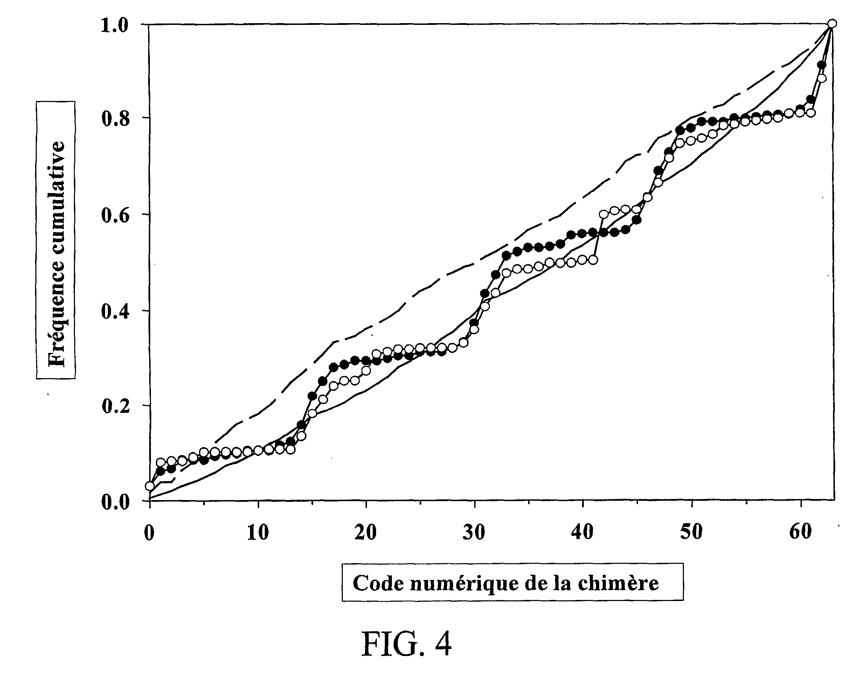

Figure 4 : Fréquences cumulées expérimentales et théoriques pour l'observation des 64 types de structures mosaïques possibles. L'axe horizontal correspond à un codage des structure mosaïques en utilisant N = P1 + 2*P2 + 4*P3 + 8*P4 + 16*P5 + 32*P6, où P1 à P6 ont les valeurs de 0 ou 1 dépendant respectivement de l'hybridation avec les séquences 1A1 ou 1A2. Les cercles ouverts représentent les courbes expérimentales déduites des états d'hybridation de la grille de 384 clones avec les six sondes oligonucléotides. La courbe continue correspond à des courbes théoriques en considérant une proportion homogène de 0,56:0,44 pour les séquences parentales 1A2 et 1A1 parental séquences et un mélange parfait (absence de corrélation croisée). La courbe en pointillée représente la même courbe pour une proportion de 50:50 pour les séquences parentales 1A1 et 1A2. Les cercles noirs représentent la courbe théorique obtenue par des simulations en considérant une proportion homogène de 0.56: 0.44 pour les séquences parentales lA2 et 1A1 mais une probabilité de liaisons parentales de 0,1 : 0,6 : 0,85 : 0,1 : 0,1 entre les segments sondés 1-2, 2-3, 3-4, 4-5 et 5-6 respectivement. La liaison est définie comme suit: 0 correspond à l'indépendance et 1 à une liaison totale.

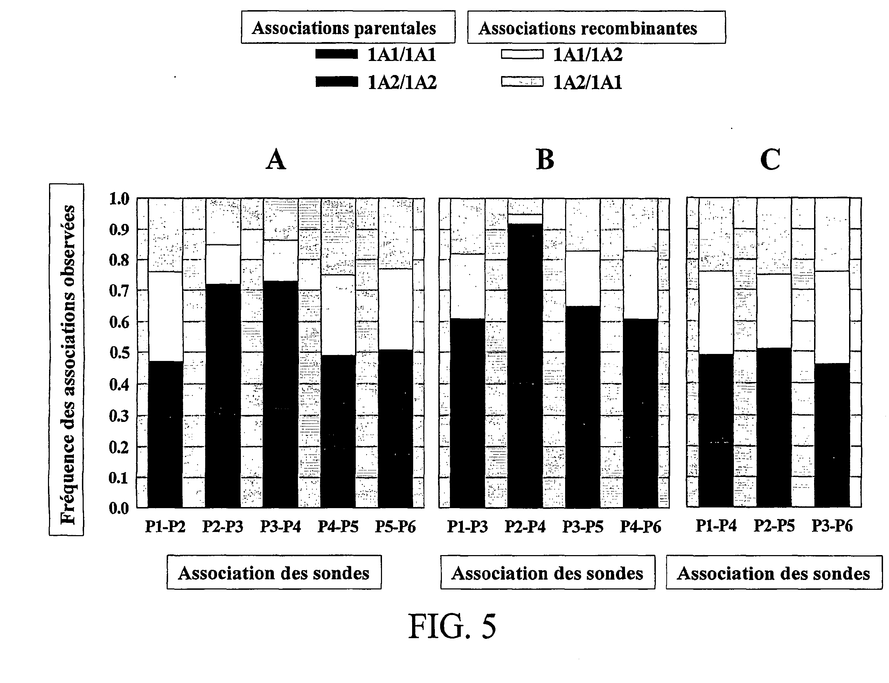

Figure 5 : Représentation des fréquences parentales et recombinantes pour l'association entre deux sondes. La fréquence de chaque association a été déterminée par une des macros générée dans Microsoft Excel. La somme des quatre différentes fréquences (parentales et recombinantes) est toujours 1. A : association entre deux sondes adjacentes ; B : association entre sondes séparées par une sonde; C : association entre sondes distantes (séparées par deux ou trois sondes). Les histogrammes noirs et gris foncés représentent les associations parentales alors que le gris clair et le gris semi-foncé représentent les associations recombinantes.

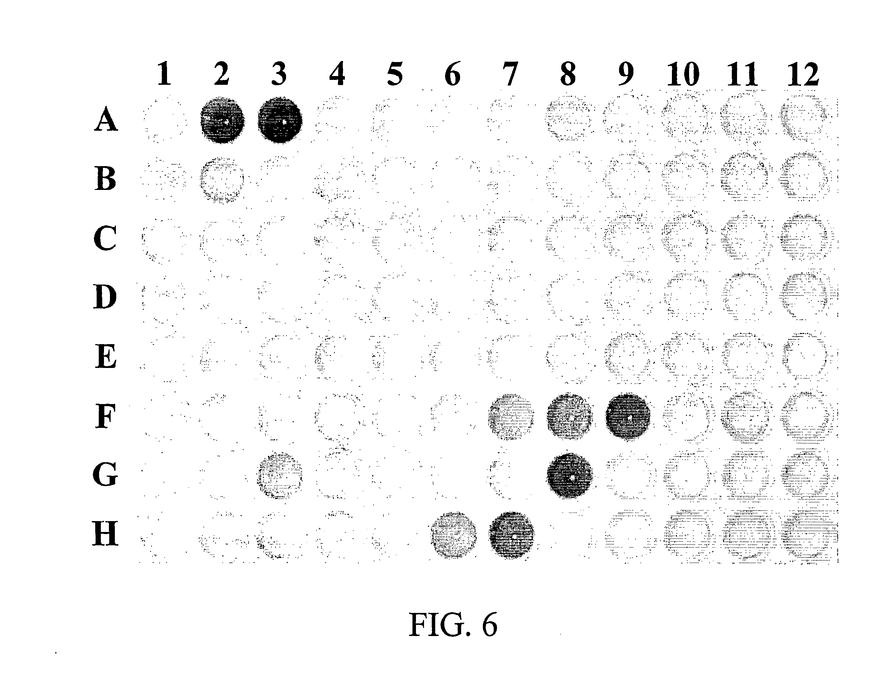

Figure 6 : Détection colorimétrique de structures mosaïques fonctionnellement compétentes pour l'oxydation du naphtalène. La bioconversion est réalisée dans 1 ml de culture de levures en présence de 1,6 mM naphtalène. L'extraction en phase solide et le développement de la coloration est entièrement réalisée dans des plaques à microtitration comme décrit dans les Exemples. La coloration foncée indique les clones positifs.

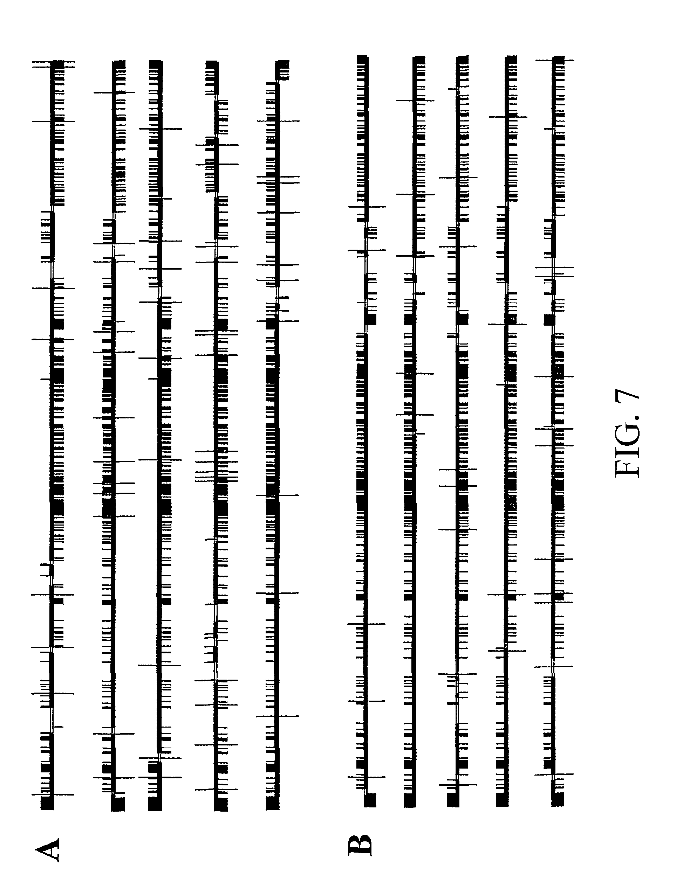

Figure 7 : Représentation schématique des séquences de 10 structures mosaïques sélectionnées au hasard : A dans la population totale; B dans la sous-population de clones actifs. Pour chaque structure un alignement nucléotidique a été réalisé avec les deux séquences parentales. Ces alignements ont été utilisés comme données de départ pour un programme d'analyse de séquences et un programme de visualisation qui a généré la figure. Les zones grises et noires correspondent respectivement à des séquences appartenant aux P450 parentaux 1A1 ou 1A2. Les traits verticaux fins inférieurs ou supérieurs indiquent les zones de misappariement nucléotidiques avec la seconde structure parentale. Les marques traversant les séquences indiquent les positions de séquences qui n'apparient avec aucune des deux séquences parentales et devant donc correspondre à des mutations. Les parties horizontales transparentes correspondent à des segments de séquences pour lesquelles l'appartenance à l'un ou l'autre des type parentaux n'a pu être déterminée par l'analyse de séquences.

### EXEMPLES

### Exemple 1 : Méthodes

### 1.A : Souches, plasmides et biologie moléculaire

Deux souches de *S. cerevisiae* ont été utilisées : W303-1B, également appelée W(N) (Mat a ; *ade2-1 ; his3, leu2, ura3, trpl, can^{R}, cyr*⁺*),* et W(R) qui dérive de W(N) par l'insertion du promoteur inductible *GAL10-CYC1* en amont de la P450 réductase endogène *(YRED)* de levure. Cette souche a été précédemment décrite par Truan et col. (40) et dans le brevet EP 595 948, incorporés ici par référence.

La souche d'*E*. *coli* utilisée a été DHS-1 (F⁻, recAl, gyrA96, thi-1, hisR17, supE44, λ⁻). Les vecteurs d'expression utilisés ont été p1A1/V60 (42) et p1A2N60 (43, incorporé ici par référence) ; ces deux vecteurs ont été construites par insertion des ORFs des *CYP1A1* et *CYP1A2* humain entre les sites de restrictions *BamHl*/*KpnI* et *BainHI*/*EcoR.I* de pYeDP60 respectivement. Ces deux vecteurs d'expression contiennent également *URA3* et *ADE2* comme marqueur de sélection et place les phases ouvertes de lecture (ORFs) sous le contrôle du promoteur *GAL10-CYC1* et du terminateur *PGK* (39, incorporé ici par référence). Tous les milieux utilisés ont été décris précédemment dans des documents incorporés ici par référence (40, 42).

Les bactéries DH5-1 ont été rendues électrocompétentes selon le protocole décrit par Sambrook *et al.* (44) incorporé ici par référence, et les cellules transformées en suivant les recommandations du constructeur de l'électroporateur (Biorad). Les cellules ont été sélectionnées sur des milieux LB solides contenant 50 µg/ml d'ampicilline.

### Transformation de levure

Après une préculture de 12 heures dans 5 ml de milieu YPGA (pour la souche W(N)) ou YPLA (pour la souche W(R)), les cellules ont été diluées dans 50 ml de milieu YPGA pour obtenir une densité finale de 2.10⁶ cellules/ml. Six heures plus tard, les cellules ont été lavées deux fois avec de l'eau stérile et une fois avec du tampon TE-lithium acétate (10mM Tris-HCl pH 7,5, 1mM EDTA, 100 mM lithium acétate). Les cellules sont ensuite resuspendues dans 1 ml de tampon TE-lithium acétate.

L'ADN transformant a ensuite été ajouté à 50 µl de la solution de cellules précédemment obtenue, ainsi que 50 µg d'ADN de sperme de saumon (préalablement soniqué et dénaturé à 95°C), et 350 µl d'une solution de PEG 4000 à 40% (w/v). Cette solution a ensuite été incubée à 30°C pendant 30 minutes et soumise à un choc thermique à 42°C pendant 45 minutes. Après centrifugation, le surnageant a été éliminés et les cellules resupendues dans 200µl d'une solution à 0,1M NaCl. Les cellules sont ensuite sélectionnées sur un milieu SWA6 solide (39, 42, incorporés ici par référence).

### Extraction de l'ADNplasmidique de levure

Les colonies sont resuspendues dans 1 ml de tampon A contenant 2‰ (v/v) de triton X-100, 50 mM de Tris-HCl pH 8,0, 50 mM d'EDTA et 200 mM de NaCl. Puis, 1 volume de billes de verre (Braun Scientifics, diamètre 0,45 mm) a été ajouté et la solution vigoureusement vortexée pendant 2 min avec 300 µl d'un mélange phénol/chloroforme/ alcool isoamylique (50:49:1, en vol.). Après récupération de la phase aqueuse, l'ADN a été précipité à l'éthanol et resuspendu dans 50 µl d'eau.

### Séquences

Cinq clones bactériens issus de la banque initiale et cinq clones fonctionnels ont été aléatoirement sélectionnés et séquencés. Les séquences ont été réalisées soit par ESGS (ESGS, group Cybergene, Evry France) ou en utilisant le kit ABI et le séquenceur ABI en suivant les protocoles du constructeur (Perkin Elmer).

### 1.B : Mélange recombinatoire d'ADN basé sur la PCR modifiée

La technique utilisée est dérivée de celle décrite par Stemmer (2, 3, 15), incorporés ici par référence. La fragmentation aléatoire avec la DNase 1 (Grade II, Sigma-Aldrich) en présence de Mn²⁺ et réalisé avec les modifications décrites par Lorimer et Pastan (45) et Zhao (46), incorporés ici par référence.

2,5 µg de chaque ADN plasmidique (plAl/V60 et plA2/V60) ont été resuspendu séparément dans un tampon contenant 50 mM de Tris-HCl pH 7,4, 10 mM de MnCl₂ pour un volume final de 40 µl. La DNase 1 a été ajoutée à trois différentes concentrations (0,0112 U/µg d'ADN, 0,0056 U/µg d'ADN et 0,0028 U/µg d'ADN). La digestion a été réalisée à 20°C pendant 10 min et la DNAse 1 inactivée en chauffant à 90 °C pendant 10 min. Les fragments obtenus ont été purifiés sur une colonne Centrisep (Princeton Separation Inc. , Philadelphia, NJ).

Pendant la réaction de réassemblage, les fragments purifiés (10 µl de chaque plasmide fragmenté) ont été amplifiés par une réaction de PCR dans 40 µl utilisant 2,5 U de Taq-polymérase (Stratagene).

Le programme de PCR utilisé consistait en : 1 cycle de dénaturation à 96°C, pendant 1.5 min ; 35 cycles de (30s de dénaturation à 94°C, 9 différentes étapes d'hybridation séparées de 3°C en allant de 65°C à 41°C et de 1,5 min chacune et une étape d'élongation de 1.5 min à 72°C) et finalement 7 min à 72°C.

La seconde réaction d'amplification a été réalisée avec un primer 5' localisé dans le promoteur *GAL10-CYC1* (SEQ ID N° 1) et un primer 3' localisé dans le terminateur PGK (SEQ ID N° 2).

### 1.C : Construction et caractérisation de la banque

Les produits d'amplification par PCR ont été séparés par gel d'électrophorèse puis purifié. Les ADNs ont été insérés dans pYeDP60 en utilisant la recombinaison *in vivo* (gap-repair) dans la levure (37, 38, 43, 47, 48). La cotransformation de la souche W303-IB avec 1/20^{e} du produit de PCR (insert) et 0,025 µg de pYeDP60 préalablement linéarisé en utilisant les enzymes de restriction *EcoRI* et *BamHI* a été réalisée.

L'ADN extrait de la levure a été utilisé pour transformer la souche d'E. *coli* DH5-1 en utilisant la résistance à l'ampicilline apportée par le plasmide. 378 puits d'une plaque à microtitratrion 384 puits ont été inoculés avec des colonies bactériennes indépendantes choisies au hasard dans la banque, 3 puits avec des bactéries DH5-1 préalablement transformé avec p1A1/V60 et les 3 puits restants avec des DHS-1 transformées par plA2/V60. Après 24 heures de croissance dans du milieu TB (44) contenant 100 µg/ml ampicilline, les 384 puits ont ensuite été répliqués sur six membranes de Nylon N+ (Amersham). Chaque filtre a été placé sur un milieu LB solide contenant 100 µg/ml d'ampicilline. Après 12 heures de croissance, la lyse des colonies bactériennes, la fixation et la dénaturation de l'ADN, la préhybridation des filtres ont été réalisés selon le protocole préconisé par le fabricant (Amersham).

11 pmoles d'oligonucléotides ont été ajoutées à 3,3 pmoles de γ-ATP marqués au ³²P, à 2 µl de polynucléotide kinase et 18 µl de tampon (New England Biolabs). L'ensemble a été incubé 2 h à température ambiante. La préhybridation des filtres a été réalisée selon le protocole préconisé par le fabricant. La sonde marquée est ajoutée dans un tube à hybridation contenant un des filtres et l'ensemble est incubé pendant 12 h à 42°C. Les filtres sont ensuite lavés dans une solution de SSPE 2x / 0,1% de SDS pendant 10 min. Les filtres ont été analysés par autoradiographie, selon un protocole connu.

Chaque sonde a été marquée une seconde fois et hybridée à un filtre différent pour s'assurer de la reproductibilité des résultats.

### 1.D : Sélection des clones contenant des P450s fonctionnels

Les colonies bactériennes ont poussé pendant 24 heures dans des plaques à microtitration 96 puits. L'extraction d'ADN a été réalisée en utilisant le protocole de l'appareil Multiscreen de minipréparation d'ADN par filtration en microplaques 96-puits (Millipore). Chaque ADN purifié a été utilisé pour transformer la souche de levure W(R) en plaque à microtitration 96 puits et les cellules ont été sélectionnées sur des milieux SWA6 solides.

Après 3 jours de croissance à 30°C, 1 ml de milieu liquide SWAS a été ensemencé avec un aliquote de chaque colonie dans une microplaque 96 puits Deepwell (ABGene) pendant 15 heures. Le milieu a été ensuite éliminé et remplacé par 1 ml de, milieu YPLA contenant 1,6 mM de naphtalène (Merck).

Pour chaque culture, le milieu de culture a ensuite été placé dans les puits correspondant d'une microplaque 96 puits Multiscreen (MABV N12, Millipore) contenant 90 µl de résine C18 sillicagel octadecyl fonctionnalisée (Aldrich). Après une filtration sous vide du milieu de culture, le substrat et les produits de la réaction sont liés à la silice. La résine a ensuite été lavée 2 fois à l'eau et les métabolites élués avec 50 µl d'isopropanol. Après addition de 20 µl d'une solution de Diazo-Blue-B à 2 mg/ml (Fluka) la réaction colorée générée par le couplage entre les précurseurs de diazo et les phénols extraits du milieu de culture a été observée.

### 1.E : Analyses statistiques

Pour chaque sonde, une grille représentant les intensités d'hybridations des 384 clones a été construite. Les intensités d'hybridations ont été analysées visuellement en tenant compte du bruit de fond environnant. Les spots excédant très largement le bruit de fond local des spots négatifs ont été considérés comme positifs, même s'ils étaient moins intenses que les spots les plus positifs. Ces réponses intermédiaires pouvant être dues à un misappariement partiel de la sonde (consécutif des étapes de PCR) ou alors à une efficacité de transfert moins importante de certains spots sur le filtre. Les ambiguïtés ont été levées par la réalisation d'hybridation d'un autre filtre par la même sonde.

Les six grilles de 384 puits ont été entrées dans des tableurs Microsoft Excel et une analyse statistique a été réalisée avec des macros Excel écrites en Microsoft Visual Basic, et reprenant les étapes d'analyse telles que décrites dans la description. Le programme converti d'abord les signaux d'hybridations en données d'un type parental par un système de masques avec une fonction booléenne XOR avant l'analyse statistique. Les analyses statistiques ont été réalisées en suivant les étapes énumérées dans la description.

Des simulations numériques ont été réalisées en utilisant un générateur de nombres aléatoires et des routines de calculs de probabilité. Le programme peut être ajusté pour simuler tous les biais possibles dans la probabilité de trouver l'un ou l'autre des types parentaux pour les zones de séquences correspondant à chacune des sondes, ainsi que toutes les «liaisons» possibles entre segments adjacents ou distants. Un premier jeu de paramètres a permis de moduler les probabilités relatives de trouver l'un ou l'autre des types parentaux pour chaque zones de séquence sondée. Un second jeu de paramètres a permis d'introduire d'une (ou plusieurs) liaison génétique entre deux fragments (ou plus) de séquence (correspondant à deux ou plusieurs sondes).

Les programmes de simulations et d'analyses statistiques ont été utilisés pour générer des grilles correspondant à différentes situations de liaisons entre fragments. Dans tous les tests, les résultats des analyses statistiques ont été en accord avec les paramètres entrés dans le programme de simulations. La méthode d'association de ces techniques de simulation et d'analyses a été également utilisée pour déterminer les fluctuations statistiques sur les données en réalisant des analyses de 10 cycles répétés de simulations et d'analyses pour chaque jeu de paramètres. Le générateur de nombres aléatoires a été réinitialisé entre chaque simulation pour en faire des événements indépendants.

### Exemple 2 : Construction d'une banque d'expression par mélange recombinatoire d'ADN d'une même famille

Le principe de la stratégie utilisée est décrit en Figure 1 : il associe une étape de mélange recombinatoire d'ADN *in vitro* par PCR modifiée à une seconde étape de mélange recombinatoire *in vivo* par recombinaison dans la levure. Cette dernière étape a également été utilisée comme un outil de clonage efficace. Ceci constitue une stratégie de shuffling complète permettant l'expression dans une cellule eucaryote et la sélection fonctionnelle sans avoir besoin d'étape de clonage intermédiaire dans *E*. *coli.*

La première étape (Figure 1) consiste en une fragmentation double brin du plasmide entier à la DNAse 1 conduisant à des fragments d'ADN de petites tailles (Figure 1A).

Les résultats de la fragmentation des plasmides p1A1/V60 et p1A2/V60 (figure 1A, lignes 2 et 5; 3 et 6; 4 et 7) ont été mélangés en proportion équimolaire et soumis à un programme de PCR original « d'hybridations progressives » (voir Exemple 1) impliquant 9 étapes d'hybridation allant de 61°C à 41 °C pour forcer la recombinaison entre fragments ayant peu d'homologies. Comme montré sur la figure 1B, dans de telles conditions, une large trace (smear) d'ADN de haut poids moléculaire a été formé quels que soient les fragments pris au départ.

Bien que ce matériel se soit trouvé avoir des propriétés de transformation directe de la levure due à une recombinaison entre fragments *in vivo* et à la reconstitution de vecteurs de levure complets et fonctionnels (11 kb) (résultats non montrés), une nouvelle étape de PCR, utilisant des amorces situées sur les séquences d'ADNc flanquantes d'initiation de la transcription CYC1 et les séquences de terminaison de la transcription PGK, a été nécessaire pour obtenir une banque de taille raisonnable (Figure 1C, lignes 5, 6 et 7). Cette dernière étape a résulté en une amplification de bande d'ADN bien définie d'environ 1,9 kb comprenant l'ADNc «mélangé-recombiné» et les régions flanquantes du vecteur.

Le produit de PCR montré en Figure 1C, ligne 6 a été utilisé pour cotransformer la levure avec pYeDP60 linéarisé au site d'expression pour utiliser les propriétés de recombinaison homologues (gap-repair) de la levure.

La cotransformation de la banque de l'ADNc de bonne taille dans la levure et du vecteur linéarisé a amené à une séries d'évènements de recombinaison déjà observé lors d'expérience précédentes de recombinaison homéologues ou gap-repair (37, 38, 43). La sélection a été basée seulement sur la recircularisation du vecteur après un ou plusieurs événements de recombinaison. Les expériences ont donné approximativement 10,000 clones.

La plupart des clones de levure étaient transformés par plusieurs plasmides. En effet, une population hétérogène de plasmides a été observée après extraction d'ADN d'une seule colonie de levure, la transformation d'*E*. *coli* et la ségrégation des clones.

Ceci permet d'évaluer la complexité de la banque initiale à entre 25,000 et 100,000 structures mosaïques pour une seule expérience de transformation de levure. La banque est utilisable comme telle pour la sélection fonctionnelle.

Des expériences similaires utilisant des fragments ADN de plus bas poids moléculaires (moins de 100 pb) comme décrit en Figure 1A, lignes 1 et 5 ont aussi conduit à une banque exploitable mais avec une efficacité inférieure. Les ADNs de plus haut poids moléculaire (Figure 1A, lignes 4 et 7) n'ont pas été utilisé pour la construction d'une banque à cause des possibilités d'un fort taux de contaminations par des structures parentales.

### Exemple 3 : Analyse statistique d'une sous-population de la banque

L'ADN plasmidique a été préparé à partir de la banque de levure et utilisé pour transformer *E*. *coli* en utilisant le marqueur de résistance à l'ampicilline présent sur le plasmide de levure. Cette étape a permis la ségrégation de plasmides individuels qui étaient initialement présents comme une population hétérogène dans chaque colonie de levure. Une matrice a été construite à partir d'une plaque de microtitration de 384-puits contenant 378 clones d'E. coli choisis au hasard pour des analyses de structures en utilisant 6 sondes réparties le long de la séquence des P450s parentaux décrites sur la figure 2 (SEQ m N° 3 à SEQ ID N° 8). Les puits restants ont été ensemencés avec des bactéries préalablement transformées avec des plasmides contrôles contenant l'une ou l'autre des séquences parentales (P450 1A1 ou 1A2).

Les six sondes (22-36 bases) ont été choisies pour hybrider alternativement sur les deux séquences parentales dans des régions de faibles similarités de séquences entre les deux P450s parentaux : 3 sondes appartenaient au p1A1/V60 et 3 au p1A2/V60. Chaque sonde a été marquée au ³²P et utilisé pour hybrider les répliques sur filtres (dans des conditions favorisant les hybridations spécifiques). Les expériences ont été répétées en utilisant différentes associations de filtres et de sondes pour éliminer d'éventuels artefacts. L'analyse des intensités d'hybridation a été réalisée manuellement. Les niveaux intermédiaires d'intensité d'hybridation (de l'ordre de 15 % des spots) ont été considérés comme des réponses positives. Ces réponses doivent correspondre à des misappariements d'une paire de base dus à des mutations induites par les différentes étapes de PCR (ceci étant confirmé par les données de séquençage (voir plus tard)) ou alors à des différences d'efficacité de transfert d'ADN.

La figure 3 présente le pattern global d'hybridation pour les six sondes. La fréquence de structures présentant un pattern d'hybridation semblable à l'un des parents (ci-après dénommés « parentaux ») pour toutes les sondes calculées dans la banque (figure 3A, carrés foncés) est de 11,4 % pour des structures correspondant au P450 1A2 et de 2,4% pour des structures correspondant au P450 1A1. La somme de ces deux fréquences (13,8%) est supérieure à la valeur théorique de 3,1% ((0,5)⁶+(0,5)⁶) correspondant à une réassociation totalement aléatoire des fragments de séquences parentales. Une illustration en "fausses couleurs" des différentes structures mosaïques (non montrée) illustre bien l'excès de clones parentaux de type 1A2 ou de type 1A1 mais suggère une répartition générale assez homogène des différents types de structures mosaïques.

Dans le but d'aller plus loin dans la caractérisation de la population, une analyse statistique a été réalisée en utilisant un programme basé sur des tableurs Excell et des routines en Visual Basic. La probabilité de présence de chaque séquence parentale à chacune des 6 positions sondées a été calculée (Tableau 1). Cette fréquence a été assez homogène (0,56 ± 0.02 pour les fragments de type 1A2) pour l'ensemble des segments analysés. Le petit excès dans la fréquence pour les segments de type 1A2 reflète probablement l'erreur dans l'évaluation des taux d'ADN parentaux lors du mélange des fragments d'ADN parentaux. La proportion théorique des séquences parentales a été recalculée avec les nouvelles valeurs de fréquence: 3,7% (0,58⁶ + 0,42⁶). Cette dernière valeur ne correspond toujours pas à la proportion de parentaux observées (13,8%).

**Tableau 1: fréquence des parties de séquences mosaïques appartenant à chaque type parental, aux positions sondées. Les sondes P1 à P6 commencent aux positions respectives des séquences de P450 1A1 ou 1A2, selon la sonde considérée : 3, 612, 683, 879, 1377 et 1513 (voir la figure 2). Pour chaque sonde, le nombre de signaux d'hybridation relatif à 1A1 ou à 1A2 a été calculé et divisé par le nombre total de clones testés (378).**

| Sonde | Fréquence du type 1A1 | Fréquence du type 1A2 |
|---|---|---|
| P1 | 0,48 | 0,52 |
| P2 | 0,43 | 0,57 |
| P3 | 0,45 | 0,55 |
| P4 | 0,45 | 0,55 |
| P5 | 0,44 | 0,56 |
| P6 | 0,41 | 0,59 |
| Moyenne ± D. S. | 0,43 ± 0,02 | 0,56 ± 0,02 |

Pour caractériser plus en détail la population la courbe des fréquences cumulées pour la probabilité d'observation des 64 classes détectables de chimères a été calculée (figure 5). Un code binaire associant arbitrairement une valeur de 0 ou 1 selon la nature de chaque segment (1A1 ou 1A2), pour les segments 1 à 6, pour chaque structure mosaïque a été utilisé. Les séquences parentales 1A1 et 1A2 correspondent respectivement aux codes 0 et 63. La courbe expérimentale (figure 5, cercles vides) a un aspect irrégulier comprenant cinq paliers. L'apparition de ces paliers était complètement inattendue, et imprévisible, car ne correspondant pas ce qui aurait été attendu dans le cas d'une indépendance de recombinaison entre les différents fragments.

Trois courbes théoriques ont alors été calculées comme décrit dans l'Exemple 1 en utilisant des approches de type Monte Carlo (simulations numériques) en utilisant différentes hypothèses :
(i) une probabilité égale de trouver les différents types parentaux aux zones de séquences correspondant aux différentes sondes et une indépendance totale de la nature de chaque segment de séquence,
(ii) hypothèse (i) mais avec une probabilité de trouver des fragments de type 1A2 aux zones de séquences correspondant aux différentes sondes de 55,8% ;
(iii) hypothèse (ii) mais la probabilité de mélange recombinatoire entre les différents segments de séquence n'est plus infinie (mélange imparfait), mais avec des liaisons variables entre la nature de segments consécutifs.

La courbe de fréquence cumulée (figure 5) correspondant à l'hypothèse (i) est linéaire alors que dans le cas correspondant à l'hypothèse (ii) la courbe est arrondie mais reste régulière. Cette courbe, (reflétant le réel pourcentage de fragments parentaux) reproduit effectivement bien l'allure générale de la courbe calculée à partir des résultats expérimentaux, mais elle ne présente pas les paliers observés.

De nombreuses courbes correspondant à l'hypothèse (iii) ont été générées avec différents types de liaisons entre segments et une courbe correspondant à la courbe expérimentale a été trouvée (cercles pleins). L'addition de liaisons génétiques appropriées entre les séquences sondées permet de déterminer une courbe correspondant qui suit la courbe expérimentale. Bien sûr, plusieurs solutions doivent ici être possibles, mais une probabilité de liaison entre fragments parentaux de 0,1 ; 0,6 ; 0,85 ; 0,1 ; 0,1 entre les segments sondés 1-2, 2-3, 3-4, 4-5 et 5-6 respectivement, donne un résultat satisfaisant. Ces résultats suggèrent que, même si la proportion de chaque type parental le long de la séquence est homogène, la probabilité de mélange recombinatoire dépend du segment de séquence considéré. Ainsi, les paliers de la courbe de résultats obtenue correspondent à une corrélation entre différents segments de séquence.

Le calcul des fréquences de chaque type parental dans la population a été simulé après incorporation des probabilités de liaisons dans le modèle. Les résultats moyens résultant de 10 simulations informatiques donnent une fréquence de structures de type parental de 13,9 ± 1.3 % (dont 9,8 ± 1.4 % pour le 1A2 et 4,1 ± 1.09 % pour 1A1), ce qui correspond assez bien aux valeurs expérimentales de 13,8 %; (11,4 % pour le 1A2 et 2,4 % pour le 1A1). L'hétérogénéité de la probabilité de mélange recombinatoire le long de la séquence peut donc parfaitement être responsable de l'excès apparent de structures de type parental dans la population.

Afin de vérifier l'existence de liaisons entre fragments, les associations entre les différentes sondes ont été analysées. La figure 6 présente les fréquences des associations de zones de séquence de même type parental et de type parental différents pour chacune des associations de sondes possibles.

Dans la figure 6.A, il est possible de voir les probabilité d'associations proches (entre zones adjacentes). Ceci met clairement en évidence que les associations P1-P2, P4-P5 et P5-P6 montrent une indépendance complète à la différence des associations P2-P3 et P3-P4 qui montrent une diminution de la fréquence d'association entre fragments de type parental différents.

La figure 6.B montre l'association entre deux sondes espacées d'une sonde. De nouveau on peut observer une association montrant une liaison presque complète entre P2 et P4. Les autres associations présentent des indépendances complètes entre sondes.

Ceci est également vrai pour des associations entre sondes plus éloignées (figure 6.C). D'autres associations à longues distances (Pl-P5; P2-P6 and P1-P6) ont été calculées, révèlent les mêmes caractéristiques que celles de la figure 6.C et ne sont pas montrées ici.

Ces résultats confirment bien le modèle prédictif même si le nombre de liaisons dans le modèle est de seulement 2. De manière surprenante les valeurs obtenues pour ces données ne correspondent pas à un modèle génétique. En effet, la distance (entre les segments liés) semble plus importante dans le cas de P2-P4 comparé à P2-P3 ou P3-P4. Une explication possible de ce phénomène peut être liée au nombre possible de crossing-over dans cet intervalle (P2-P4).

L'existence de paliers correspondant à une corrélation entre fragments, lorsque l'on utilise l'analyse décrite plus haut permet de tirer une conclusion importante. En effet, lorsqu'une pression de sélection fonctionnelle est exercée sur les clones, il est probable qu'elle introduira un biais plus important de corrélations entre différentes régions des gènes étudiés. Ainsi, il peut être possible de définir des patterns d'association entre plusieurs régions du gène, qui sont liés à des activités et/ou propriétés fonctionnelles. Ceci doit permettre d'accélérer le processus de définition de protéines présentant des fonctions et/ou propriétés améliorées, en choisissant les séquences à associer.

### Exemple 4 : Sélection de clones fonctionnels

Un avantage majeur de la stratégie de mélange recombinatoire (shuffling) développée dans la présente invention est que la banque est, pour la première fois, directement construite dans un microorganisme eucaryote (la levure). Il est de plus possible d'utiliser des souches de levure dont le génome a été modifié afin de permettre de reconstituer des systèmes protéiques (enzymatiques) complexes.

Dans les expériences de la présente invention, des souches de levures possédant un génome modifié ont été utilisées, pour permettre la reconstitution d'un système membranaire avec couplage des différents partenaires. Les clones de levure transformés résultant des étapes de shuffling peuvent alors être utilisés en tant que tels pour un criblage fonctionnel de l'activité des protéines mosaïques construites.

L'utilisation de la banque primaire offre de plus l'avantage d'être constituée de clones contenant de multiples plasmides mosaïques ce qui améliore considérablement la complexité de la banque, et permet de cribler les activités de plusieurs protéines mosaïques en testant l'activité sur juste un clone de levure.

Cependant, il est clair que les clones sélectionnés pour leur fonctionnalité nécessitent une étape de ségrégation supplémentaire pour une étude biochimique plus détaillée. Cette ségrégation peut-être réalisée par des sous-clonages répétés ou par des extractions d'ADN des clones positifs, suivis d'un transfert dans E. *coli* et d'une retransformation de la levure.

Les expériences suivantes démontrent la faisabilité d'une sélection fonctionnelle directe *in vivo* dans des plaques de microtitration.

La méthode est basée sur une technique universelle de détection par coloration des phénols aromatiques formés par bioconversion *in vivo* directe des hydrocarbures polycycliques aromatiques dans des cultures en microplaques 96-puits (voir Exemple 1).

Les dérivés phénoliques ont ensuite été extraits par fixations hydrophobes (sur des résines de C18) directement sur des microplaques et révélés par colorimétrie consécutive au couplage avec des précurseurs de diazo-fast dyes (Figure 7).

Le criblage de la banque mosaïque 1A1 / 1A2 a été réalisé en utilisant le naphtalène qui est un bon substrat des deux enzymes parentales. Dans le but de déterminer la réelle proportion de structures fonctionnelles la banque primaire dans la levure a été transférée dans *E. coli* et 96 clones indépendant (et donc ne contenant qu'un type de plasmide) ont été utilisés pour retransformer la levure dans des plaques à microtitration. La fréquence de clones fonctionnels dans de telles conditions (12 % pour la librairie construite avec la Taq DNA polymérase) a été reconfirmée par des méthodes classiques utilisant des analyses des produits extraits par HPLC.

Ces contrôles ont permis de voir que la détection colorimétrique est fiable et a une sensibilité suffisante pour détecter des clones avec une activité naphtalène hydroxylase représentant seulement 10 % de l'activité parentale (ces différences de quantités de métabolites produits pouvant être dues à des différences d'activités mais également d'expression des enzymes mosaïques).

La méthode de détection utilisée s'est également révélée être efficace pour la détection de métabolites issus de du métabolisme du phénanthrène ou d'autres hydrocarbures polycycliques aromatiques.

### Exemple 5 : Analyses de séquences de la banque

Cinq clones sélectionnés au hasard indépendamment de critères fonctionnels et les cinq clones choisis dans la sous-population de clones fonctionnels (voir plus loin pour la sélection) ont été séquencés. Ces structures se sont avérées être des mosaïques contenant, de plus, des mutations additionnelles.

Les structures mosaïques sont décrites en figure 7. La figure est basée sur un alignement entre les structures mosaïques et les deux séquences parentales et a été réalisée avec l'aide d'un logiciel approprié : pour chaque structure, un alignement nucléotidique a été réalisé avec les deux séquences parentales. Ces alignements ont été utilisés comme données de départ pour un programme de visualisation qui a généré la figure, en dessinant en gris ou en noir les parties de séquences appartenant respectivement aux P450 parentaux 1A1 ou 1A2, et en ajoutant des traits verticaux fins inférieurs ou supérieurs pour indiquer les zones de misappariement nucléotidiques avec la seconde structure parentale. Par ailleurs, des traits traversant les séquences indiquent les positions de séquences qui n'apparient avec aucune des deux séquences parentales et devant donc correspondre à des mutations. Le logiciel dessine également des parties horizontales transparentes, qui correspondent à des segments de séquences pour lesquelles l'appartenance à l'un ou l'autre des type parentaux n'a pu être déterminée par l'analyse de séquences.

L'analyse de ces 10 séquences sélectionnées au hasard confirme la présence de structures mosaïques pour chaque séquence. En analysant l'ensemble de ces structures on peut noter un nombre moyen de fragments différents de 5,4 ± 2,2. La distribution de taille de ces fragments est homogène. Pour les 54 fragments considérés, 32 ont des tailles comprises entre 0 et 200 bp, 12 entre 200 et 500 bp et 10 entre 500 et 1000 bp. De plus, environ 60 % des fragments sont de taille inférieure à 200 bp, la taille du plus petit fragment échangé étant approximativement de 20 bp. Ces résultats sont en accord avec la taille moyenne des fragments de départ issus de la fragmentation à la DNase 1 (200-300 bp, voir figure 1A).

L'analyse de l'activité naphtalène hydroxylase des 5 clones pris au hasard a montré que seul un était actif (clone A1). Par la suite il a été considéré comme un clone actif, au même titre que les 5 choisis sur des critères d'activité. Le taux moyen de mutations par séquence a été calculé pour les clones actifs et inactifs. Pour les clones inactifs (A2, A3, A4, A5), le nombre moyen de mutations est 14,0 (± 4,2). pour les clones actifs il est inférieur (8,3 ± 3,2). Ceci n'est pas surprenant à cause du mode de sélection (activité). En effet, les séquences des clones inactifs peuvent contenir des codons stop précoces.

Finalement, les différents résultats observés lors des analyses statistiques ont été confirmés par les données de séquences. De plus, même si le nombre de clones séquencés est faible (10), les données obtenues fournissent une vue détaillée de quelques structures mosaïques. La liaison entre fragments observée (entre les sondes 2, 3 et 4) lors des analyses statistiques est également observée dans ces séquences. En effet, on n'observe pas d'échange de fragments dans la partie centrale correspondant auxdites sondes.

Le taux élevé de mutations est en accord avec une relativement faible proportion de structures fonctionnelles (15 %) dans la population. Cependant, des expériences similaires de mélange recombinatoire réalisées en utilisant des enzymes plus fidèles que la Taq DNA polymérase telles que la Pfu et la Dynazyme EXT DNA polymérase ont donné une plus forte proportion (80-90 %) de structures fonctionnelles. Le taux de mutations peut ainsi être adapté selon les desiderata.

Les exemples ci-dessus illustrent un aspect de l'invention et l'homme du métier sait apporter les ajustements nécessaires pour généraliser les enseignements, sans s'éloigner de l'esprit de l'invention.

### REFERENCES

1. van der Meer et al. ( 1992) Microbiological Reviews, 56(4), 677-94.
2. Stemmer, W. P. (1994) Nature, 370(6488), 389-91.
3. Stemmer, W. P. (1994) Pf-oc. Natl. Acad. Sci. USA, 91(22), 10747-51.
4. Crameri et al. (1997) Nature Biotechnology, 15(5), 436-8.
5. Zhang et al. (1997) Proc. Nalt. Acad. Sci. USA, 94(9), 4504-9.
6. Crameri et al (1996) Nature Biotechnology, 14(3), 315-9.
7. Crameri et al (1996) Nature Medicine, 2(1), 100-2.
8. Giver et Arnold (1998) Current Opinion in Chernical Biology, 2(3), 335-8.
9. Giver et al (1998) Proc. Natl. Acad Sci. USA, 95(22), 12809-13.
10. Kumamaru et al (1998) Nature Biotechnology,16(7), 663-6.
11. Moore et al (1997) J. Mol. Biol., 272(3), 336-47.
12. Moore et Arnold (1996) Nature Bioteclinology, 14(4), 45 8-67.
13. Yano et al (1998) Proc. Natl. Acad. Sci. USA, 95(10), 5511-5.
14. Harayama, S. ( 1998) Trends In Biotechnology, 16(2), 76-82.
15. Crameri et al (1998) Nature, 391(6664), 288-91.
16. Nixon et al (1998) Trends In Biotechnology, 16(6), 25 8-64.
17. Kimura et al (1997) Journal of bacteriology, 179(12), 3936-43.
18. Back, K. et Chappell, J. (1996) Proc. Natl. Acad. Sci. USA, 93, 6841-5.
19. Campbell et al (1997) Nat Biotechnol, 15(5), 43 9-43.
20. Nelson et al. (1987) In Guenguerich, F. P. (ed.), Mammalian cytochrome P-450. CRC Press, Boca Raton and Florida.s, pp. pp 19-79.
21. Harris, C. C. (1989) Carcinogenesis, 10(9), 1563-6*.*
22. Kadlubar et al. In Guenguerich, F. P. (ed.), Mammalian cytochrome P-450. CRC Press : Boca Raton and Florida.s., 1987, pp. 81-130.
23. Buters et al. (1999) Drug Metab Rev, 31(2), 437-47.
24. Kawajiri et al. (1990) Princess Takamatsu Symposia, 21, 55-61.
25. Kawajiri et al. (1990) FEBS Letters, 263(1), 131-3.
26. Kawajiri et al. (1993) Critical Reviews in Oncology-Hematology, 14, 77-87.
27. Mace et al. (1994) Molecular Carcinogenesis, 11(2), 65-73.
28. Joo et al. (1999) Chemistry & Biology, 6(10), 699-706.
29. Shao et Arnold (1996) Current Opinion in Structural Biology, 6(4), 513-8.
30. Arnold, F. H. (1998) Nature Biotechnology, 16(7), 617-8.
31. Michnick, S. W. et Arnold, F. H. (1999) Nat Biotechnol, 17(12), 1159-60.
32. Kikuchi et al. (1999) Gene, 236(1), 159-67.
33. Kikuchi et al. (2000) Gene, 243(1-2), 133-7.
34. Ostermeier et al. (1999) Nat Biotechnol,17(12), 1205-9.
35. Volkov et al. (1999) Nucleic Acids Res, 27(18), e18.
36. Okuta et al (1998) Gene, 212(2), 221-8.
37. Pompon, D. et Nicolas, A. (1989) Gene, 83(1), 15-24.
38. Mezard, C., Pompon, D. et Nicolas, A. (1992) Cell, 70(4), 659-70.
39. Cullin, C. et Pompon, D. (1988) Gene, 65(2), 203-17.
40. Truan et al ( 1993) Gene, 125(1), 49-55.
41. Pompon et al. ( 1997) J Hepatol, 26 Suppl 2, 81-5.
42. Urban et al. (1990) Biochimie, 72(6-7), 463-72.
43. Bellamine et al. (1994) Eur JBiochem, 225(3), 1005-13.
44. Sambrook et al. (1989) Molecular cloning : a laboratory manual. 2nd Ed. Cold Spring Harbor Lab., Cold Spring Harbor, New York.
45. Lorimer, I. A. et Pastan, I. (1995) Nucleic Acids Res, 23(15), 3067-8.
46. Zhao, H. et Arnold, F. H. (1997) Nucleic Acids Research, 25(6), 1307-8.
47. Pompon et al. (1996) Methods Enzymol, 272, 51-64.
48. Pompon, D. (1988) Eur J Biochem, 177(2),285-93.
49. Smith et Waterman (1981)Ad. App. Math. 2 : 482
50. Neddleman et Wunsch (1970) J Mol. Biol. 48 : 443
51. Pearson et Lipman (1988) Proc. Natl. Acad. Sci. USA 85 : 2444

## Revendications

1. Procédé de construction d'une banque combinatoire d'expression fonctionnelle à partir d'une banque combinatoire d'acides nucléiques appartenant à une même famille génique, comportant les étapes consistant à :
a. introduire ladite banque combinatoire d'acides nucléiques dans une levure, simultanément avec un vecteur d'expression
b. obtenir ladite banque d'expression fonctionnelle par
- recombinaison homologue de ladite banque combinatoire d'acides nucléiques avec ledit vecteur d'expression dans ladite levure, et
- recombinaison homologue ou homéologue (entre les séquences semblables mais non identiques), entre les différents acides nucléiques de la banque combinatoire introduite dans ladite levure, afin d'augmenter la complexité et la diversité de la banque combinatoire d'expression fonctionnelle obtenue,
dans lequel ladite banque combinatoire d'acides nucléiques est un mélange de produits de PCR obtenu par amplification d'une banque combinatoire de phases ouvertes de lecture, lesdites phases ouvertes de lectures présentant entre elles une identité de séquence supérieure à 40 %, en utilisant un couple d'amorces situées dans des régions flanquant lesdites phases ouvertes de lecture, ladite banque combinatoire étant obtenue à partir d'ADN homologues ou variants de séquence différant par une ou plusieurs mutations et
ladite banque combinatoire de phases ouvertes de lecture est obtenue par réassemblage par « primer extension » de produits de fragmentation d'au moins deux phases ouvertes de lectures codant pour des protéines fonctionnelles et ladite étape de réassemblage par « primer extension » est effectuée par PCR,
**caractérisé en ce que** chaque cycle de ladite étape de réassemblage par PCR présente au moins deux paliers d'hybridation.

2. Procédé selon la revendication 1, **caractérisé en ce que** lesdites régions flanquant les phases ouvertes de lecture sont des régions promotrices et terminatrices permettant l'expression chez la levure.

3. Procédé selon l'une des revendications 1 ou 2, **caractérisé en ce que** chaque cycle de ladite étape de réassamblage par PCR présente au moins quatre paliers d'hybridation de plus de 60 secondes, par températures décroissantes régulièrement espacées.

4. Procédé selon l'une des revendications 1 à 3, **caractérisé en ce que** lesdits produits de fragmentation sont obtenus à partir d'un vecteur d'expression autonome de levure de taille totale supérieure à 7 kb, incluant les phases ouvertes de lecture.

5. Procédé selon la revendication 4, **caractérisé en ce que** ledit vecteur d'expression incluant les phases ouvertes de lecture est un vecteur d'expression pour une cellule eucaryote, et navette pour la levure.

6. Procédé selon l'une des revendications 4 ou 5, **caractérisé en ce que** ledit vecteur d'expression incluant les phases ouvertes de lecture contient également les éléments nécessaires pour se répliquer de façon autonome chez *Escherichia coli.*

7. Procédé selon l'une des revendications 4, à 6, **caractérisé en ce que** ledit vecteur d'expression incluant les phases ouvertes de lecture contient une phase ouverte de lecture codant pour un enzyme membranaire eucaryote.

8. Procédé selon la revendication 7, **caractérisé en ce que** ledit enzyme eucaryote est choisi dans le groupe constitué des cytochromes P450 eucaryotes, enzymes de conjugaison eucaryotes (de phase II), membres de la famille des transporteurs ABC eucaryotes.

9. Procédé selon l'une des revendications 1 à 8, **caractérisé en ce que** ledit vecteur d'expression avec lequel est effectué la recombinaison dans la levure est linéarisé au site de clonage normal de l'ADNc et possède des séquences promotrices et terminatrices de transcription, la recombinaison s'effectuant au niveau desdites séquences.

10. Procédé selon l'une des revendications 1 à 9, **caractérisé en ce que** ledit vecteur d'expression possède également la capacité de se répliquer de façon autonome dans des cellules eucaryotes et/ou dans *Escherichia coli.*

11. Procédé selon l'une des revendications 1 à 10, **caractérisé en ce que** la souche de levure utilisée présente une modification génétique permettant la surexpression d'au moins une protéine choisie dans le groupe constitué d'une P450 réductase endogène ou exogène, une adrénodoxine, une adrénodoxine réductase, un cytochrome b5 hétérologue, une enzyme de phase II (en particulier une époxide hydrolase).

12. Procédé selon l'une des revendications 1 à 11, **caractérisé en ce que** l'étape b. est suivie des étapes consistant à :
c. extraire l'ADN plasmidique d'au moins un clone de levure,
d. transformer une souche *d'Escherichia coli* avec ledit ADN plasmidique extrait, et sélectionner les clones transformés sur milieu approprié pour obtenir une discrimination des éléments de la banque combinatoire d'expression fonctionnelle.

13. Procédé de production de protéines fonctionnelles mosaïques actives, **caractérisé en ce que** l'on construit une banque combinatoire d'expression fonctionnelle par un procédé selon l'une des revendications 1 à 12, que l'on exprime les protéines mosaïques, et que l'on sélectionne les protéines fonctionnelles mosaïques actives par l'étude de leur activité.

14. Procédé selon la revendication 13, **caractérisé en ce que** lesdites protéines fonctionnelles mosaïques actives sont dérivées d'enzymes.

15. Procédé selon la revendication 14, **caractérisé en ce que** lesdites protéines fonctionnelles mosaïques actives sont dérivées de cytochromes P450.

16. Procédé d'analyse d'une banque combinatoire d'expression fonctionnelle selon l'une des revendications 1 à 12, **caractérisé en ce qu'**il comprend les étapes suivantes :
a. préparation d'une banque combinatoire d'expression fonctionnelle par un procédé selon l'une des revendications 1-12, transformation d'une souche *d'Escherichia coli* avec l'ADN
b. plasmidique extrait de la souche de levure ou d'un pool de levures,
c. hybridation de l'ADN plasmidique contenu dans chacun des clones individuels *d'Escherichia coli* obtenus à l'issu de l'étape a. avec une ou plusieurs sondes spécifique(s) d'une séquence parentale.

17. Procédé selon la revendication 16, **caractérisé en ce que** ladite hybridation s'effectue sur un macro- ou un micro-réseau d'ADN, ledit réseau étant constitué soit de l'ADN plasmidique contenu dans chacun des clones individuels *d'Escherichia coli* obtenus à l'issu de l'étape a., ou d'un produit de PCR de celui-ci, soit desdites sondes spécifiques, attaché(es) sur un support solide, chacun des acides nucléiques étant repéré par sa position dans ledit réseau.

18. Procédé de détermination de liens entre signatures séquentielles et signatures fonctionnelles d'une protéine, **caractérisé en ce qu'**il comprend les étapes consistant à
a. préparer une banque combinatoire d'expression fonctionnelle par un procédé selon l'une des revendications 1 à 12,
b. produire les protéines fonctionnelles mosaïques actives par un procédé selon l'une des revendications 13 à 15,
c. analyser les différences fonctionnelles et/ou d'activité entre lesdites protéines mosaïques,
d. analyser les acides nucléiques correspondant auxdites protéines mosaïques par un procédé selon la revendication 16 ou 17, suivi de façon optionnelle par un procédé d'analyse d'une empreinte d'hybridation comprenant les étapes consistant à :
i. calculer la fréquence d'apparition de chacune des combinaisons obtenues à l'étape a,
ii. procéder à une analyse statistique en comparant la fréquence d'apparition calculée à l'étape i. à la fréquence d'apparition théorique pour chaque combinaison, par un traitement mathématique et statistique adéquat
e. déterminer les liens qui peuvent exister entre les structures séquentielle observées dans l'étape d. avec les différences fonctionnelles et/ou d'activité observées dans l'étape c.

19. Procédé de prédiction de structures ayant une fonction déterminée, **caractérisé en ce que** l'on met en oeuvre le procédé selon la revendication 18 pour identifier les zones de séquences, ou les liens entre les zones de séquences, reliés à ladite fonction, et que l'on en déduit la structure recherchée.

20. Procédé d'obtention d'une protéine possédant des propriétés améliorées, **caractérisé en ce qu'**il comprend les étapes consistant à :
a. construire une banque combinatoire d'expression fonctionnelle par un procédé selon l'une des revendications 1 à 12,
b. analyser ladite banque combinatoire d'expression fonctionnelle par un procédé selon l'une des revendications 16 ou 17,
c. analyser les empreintes d'hybridation obtenues dans l'étape b. par un procédé d'analyse d'empreinte d'hybridation comprenant les étapes consistant à :
i. calculer la fréquence d'apparition de chacune des combinaisons possibles obtenue à l'étape a.,
ii. procéder à une analyse statistique en comparant la fréquence d'apparition calculée à l'étape c.i. à la fréquence d'apparition théorique pour chaque combinaison, par un traitement mathématique et statistique adéquat.,
d. déterminer les liens entre les structures séquentielles et les structures fonctionnelles des protéines par comparaison desdites empreintes d'hybridation avec les propriétés des protéines mosaïques correspondantes, par un procédé selon la revendication 18,
e. prédire les structures d'intérêt ou les organisations de structures dans les protéines mosaïques par un procédé selon la revendication 19,
f. répéter les étapes a. à e., en utilisant, comme acides nucléiques de départ pour la génération de la banque combinatoire d'expression fonctionnelle, les acides nucléiques portant les structures d'intérêt ou les organisations de structures identifiées dans l'étape e., un nombre suffisant de fois pour obtenir la protéine possédant des propriétés améliorées recherchées.

21. Procédé de détermination d'une structure d'une protéine importante en réponse à une pression de sélection à partir d'une banque combinatoire d'expression fonctionnelle obtenue par un procédé selon l'une des revendications 1 à 12 et analysée selon un procédé de l'une des revendications 16 ou 17, pour les éléments de laquelle une signature a été obtenue, comprenant les étapes de:
- normaliser ladite banque en assurant que chaque signature se trouve avec la même probabilité dans la banque normalisée,
- appliquer une pression de sélection,
- procéder à une analyse statistique des fréquences des signatures de séquence de la nouvelle banque ainsi obtenue par rapport à celles de la banque normalisée de départ;
- étudier le changement des fréquences d'apparation des signatures de séquences de la nouvelle banque obtenue, par rapport à la banque de départ normalisée, en déduisant ainsi les structures présentes ou absentes en réponse à la pression de sélection.

## Claims

1. Method for constructing a combinatorial functional expression library using a combinatorial library of nucleic acids belonging to the same gene family, comprising the steps consisting in:
a. introducing said combinatorial library of nucleic acids into a yeast, simultaneously with an expression vector,
b. obtaining said functional expression library by
- homologous recombination of said combinatorial library of nucleic acids with said expression vector in said yeast, and
- homologous or homeologous (between similar but not identical sequences) recombination, between the various nucleic acids of the combinatorial library introduced into said yeast, in order to increase the complexity and diversity of the combinatorial functional expression library obtained,
in which said combinatorial library of nucleic acids is a mixture of PCR products obtained by amplifying a combinatorial open reading frame library, said open reading frames exhibiting more than 40% sequence identity with one another, using a pair of primers located in regions flanking said open reading frames, said combinatorial library being obtained from homologous or sequence variant DNAs differing by one or more mutations and said combinatorial open reading frame library is obtained by reassembly by "primer extension" of fragmentation products from at least two open reading frames encoding functional proteins and said step of reassembly by "primer extension" is carried out by PCR, **characterized in that** each cycle of said step of reassembly by PCR has at least two hybridization stages.

2. Method according to Claim 1, **characterized in that** said regions flanking the open reading frames are promoter and terminator regions which allow expression in yeast.

3. Method according to either of Claims 1 and 2, **characterized in that** each cycle of said step of reassembly by PCR has at least four hybridization stages of more than 60 seconds, with evenly spaced decreasing temperatures.

4. Method according to one of Claims 1 to 3, **characterized in that** said fragmentation products are obtained using an autonomous yeast expression vector more than 7 kb in total size, including the open reading frames.

5. Method according to Claim 4, **characterized in that** said expression vector, including the open reading frames, is an expression vector for a eukaryotic cell, and a shuttle for yeast.

6. Method according to either of Claims 4 and 5, **characterized in that** said expression vector, including the open reading frames, also contains the elements required for autonomous replication in Escherichia coli.

7. Method according to one of Claims 4 to 6, **characterized in that** said expression vector, including the open reading frames, contains an open reading frame encoding a eukaryotic membrane-bound enzyme.

8. Method according to Claim 7, **characterized in that** said eukaryotic enzyme is chosen from the group consisting of eukaryotic cytochrome P450s, eukaryotic conjugation enzymes (phase II enzymes) and members of the eukaryotic ABC transporter family.

9. Method according to one of Claims 1 to 8, **characterized in that** said expression vector with which the recombination is carried out in the yeast is linearized at the normal cDNA cloning site and has transcription promoter and termination sequences, the recombination being carried out at the level of said sequences.

10. Method according to one of Claims 1 to 9, **characterized in that** said expression vector also has the capacity to replicate autonomously in eukaryotic cells and/or in Escherichia coli.

11. Method according to one of Claims 1 to 10, **characterized in that** the yeast strain used has a genetic modification allowing the overexpression of at least one protein chosen from the group consisting of an endogenous or exogenous P450 reductase, an adrenodoxin, an adrenodoxin reductase, a heterologous cytochrome b5 and a phase II enzyme (in particular an epoxide hydrolase).

12. Method according to one of Claims 1 to 11, **characterized in that** step b. is followed by the steps consisting in:
c. extracting the plasmid DNA from at least one yeast clone,
d. transforming an Escherichia coli strain with said extracted plasmid DNA and selecting the transformed clones on suitable medium in order to discriminate between the elements of the combinatorial functional expression library.

13. Method for producing functional active mosaic proteins, **characterized in that** a combinatorial functional expression library is constructed using a method according to one of Claims 1 to 12, **in that** the mosaic proteins are expressed and **in that** the functional active mosaic proteins are selected by studying their activity.

14. Method according to Claim 13, **characterized in that** said functional active mosaic proteins are derived from enzymes.

15. Method according to Claim 14, **characterized in that** said functional active mosaic proteins are derived from cytochrome P450s.

16. Method for analyzing a combinatorial functional expression library according to one of Claims 1 to 12, **characterized in that** it comprises the following steps:
a. preparation of a combinatorial functional expression library using a method according to one of Claims 1 to 12,
b. transformation of an Escherichia coli strain with the plasmid DNA extracted from the yeast strain or from a pool of yeasts,
c. hybridization of the plasmid DNA contained in each of the individual Escherichia coli clones obtained at the end of step a. with one or more probe(s) specific for a parental sequence.

17. Method according to Claim 16, **characterized in that** said hybridization takes place on a DNA macro- or microarray, said array consisting either of the plasmid DNA contained in each of the individual Escherichia coli clones obtained at the end of step a., or a PCR product thereof, or of said specific probes, attached to a solid support, each of the nucleic acids being located via its position in said array.

18. Method for determining links between sequence signatures and functional signatures of a protein, **characterized in that** it comprises the steps consisting in
a. preparing a combinatorial functional expression library using a method according to one of Claims 1 to 12,
b. producing the functional active mosaic proteins using a method according to one of Claims 13 to 15,
c. analyzing the functional differences and/or the differences in activity between said mosaic proteins,
d. analyzing the nucleic acids corresponding to said mosaic proteins using a method according to Claim 16 or 17, optionally followed by a method for analyzing a hybridization footprint, comprising the steps consisting in:
i. calculating the frequency of appearance of each of the combinations obtained in step a.
ii. carrying out a statistical analysis by comparing the frequency of appearance calculated in step i. with the theoretical frequency of appearance for each combination, using suitable mathematical and statistical processing,
e. determining the links which may exist between the sequence structures observed in step d. to the functional differences and/or the differences in activity observed in step c.

19. Method for predicting structures which have a given function, **characterized in that** the method according to Claim 18 is implemented in order to identify the sequence regions, or the links between the sequence regions, related to said function, and **in that** the structure being sought is deduced therefrom.

20. Method for obtaining a protein having enhanced properties, **characterized in that** it comprises the steps consisting in:
a. constructing a combinatorial functional expression library using a method according to one of Claims 1 to 12,
b. analyzing said combinatorial functional expression library using a method according to either of Claims 16 and 17,
c. analyzing the hybridization footprints obtained in step b. using a method for analyzing hybridization footprints, comprising the steps consisting in:
i. calculating the frequency of appearance of each of the possible combinations obtained in step a.,
ii. carrying out a statistical analysis by comparing the frequency of appearance calculated in step c.i. with the theoretical frequency of appearance for each combination, using suitable mathematical and statistical processing,
d. determining the links between the sequence structures and functional structures of the proteins by comparing said hybridization footprints with the properties of the corresponding mosaic proteins, using a method according to Claim 18,
e. predicting the structures of interest or the structural organizations in the mosaic proteins using a method according to Claim 19,
f. repeating steps a. to e., using, as starting nucleic acids for generating the combinatorial functional expression library, the nucleic acids bearing the structures of interest or the structural organizations identified in step e., a sufficient number of times to obtain the protein having desired enhanced properties.

21. Method for determining a protein structure which is important in response to a selection pressure, using a combinatorial functional expression library which has been obtained using a method according to one of Claims 1 to 12 and analyzed according to a method of either of Claims 16 and 17, for the elements of which a signature has been obtained, comprising the steps of:
- normalizing said library, ensuring that each signature is present with the same probability in the normalized library,
- applying a selection pressure
- carrying out a statistical analysis of the frequencies of the sequence signatures of the novel library thus obtained, compared to those of the normalized starting library;
- studying the change in the frequencies of appearance of the sequence signatures of the novel library obtained, compared to the normalized starting library, thus deducing therefrom the structures which are present or absent in response to the selection pressure.

## Patentansprüche

1. Verfahren zur Konstruktion einer kombinatorischen funktionellen Expressionsbank ausgehend von einer kombinatorischen Bank von Nukleinsäuren, die zu derselben Genfamilie gehören, umfassend die folgenden Schritte:
a. Einbringen der kombinatorischen Bank von Nukleinsäuren in eine Hefe gleichzeitig mit einem Expressionsvektor,
b. Erhalten der funktionellen Expressionsbank durch
- homologe Rekombination der kombinatorischen Bank von Nukleinsäuren mit dem Expressionsvektor in der Hefe und
- homologe oder homöologe Rekombination (zwischen ähnlichen, aber nicht identischen Sequenzen) zwischen den verschiedenen Nukleinsäuren der in die Hefe eingebrachten kombinatorischen Bank, um die Komplexität und die Diversität der erhaltenen kombinatorischen funktionellen Expressionsbank zu erhöhen,
wobei die kombinatorische Bank von Nukleinsäuren ein Gemisch von PCR-Produkten ist, die erhalten wurden durch Amplifikation einer kombinatorischen Bank von offenen Leserahmen, wobei die offenen Leserahmen untereinander eine Sequenzidentität von mehr als 40% aufweisen, unter Verwendung eines Primerpaares für die flankierenden Regionen dieser offenen Leserahmen, wobei die kombinatorische Bank ausgehend von homologen DNA oder Sequenzvarianten, die sich durch eine oder mehrere Mutationen unterscheiden, erhalten wird, und
wobei die kombinatorische Bank von offenen Leserahmen erhalten wird durch Zusammenfügen mittels "Primer Extension" von Fragmentierungsprodukten von mindestens zwei offenen Leserahmen, die für funktionelle Proteine codieren, und
der Schritt des Zusammenfügens mittels "Primer Extension" durch PCR erfolgt,
**dadurch gekennzeichnet, dass** jeder Zyklus des Zusammenfügungsschrittes durch PCR mindestens zwei Hybridisierungsstufen aufweist.

2. Verfahren nach Anspruch 1, **dadurch gekennzeichnet, dass** die Regionen, welche die offenen Leserahmen flankieren, Promotor- und Terminatorregionen sind, welche die Expression in Hefe ermöglichen.

3. Verfahren nach einem der Ansprüche 1 oder 2,
**dadurch gekennzeichnet, dass** jeder Zyklus des Zusammenfügungsschrittes durch PCR mindestens vier Hybridisierungsstufen von mehr als 60 Sekunden durch in regelmäßigen Abständen abnehmende Temperaturen aufweist.

4. Verfahren nach einem der Ansprüche 1 bis 3, **dadurch gekennzeichnet, dass** die Fragmentierungsprodukte aus einem autonomen Hefe-Expressionsvektor mit einer Gesamtgröße von mehr als 7 kb erhalten werden, der die offenen Leserahmen enthält.

5. Verfahren nach Anspruch 4, **dadurch gekennzeichnet, dass** der Expressionsvektor, der die offenen Leserahmen enthält, ein Expressionsvektor für eine eukaryotische Zelle und ein Shuttlevektor für Hefe ist.

6. Verfahren nach einem der Ansprüche 4 oder 5,
**dadurch gekennzeichnet, dass** der Expressionsvektor, der die offenen Leserahmen enthält, außerdem die für seine autonome Replikation in *Escherichia coli* notwendigen Elemente enthält.

7. Verfahren nach einem der Ansprüche 4 bis 6, **dadurch gekennzeichnet, dass** der Expressionsvektor, der die offenen Leserahmen enthält, einen offenen Leserahmen enthält, der für ein eukaryotisches membranständiges Enzym codiert.

8. Verfahren nach Anspruch 7, wobei das eukaryotische Enzym aus der Gruppe ausgewählt ist, die aus eukaryotischen P450-Cytochromen, eukaryotischen (Phase-II-) Konjugationsenzymen, Mitgliedern der Familie der eukaryotischen ABC-Transporter besteht.

9. Verfahren nach einem der Ansprüche 1 bis 8, **dadurch gekennzeichnet, dass** der Expressionsvektor, mit dem die Rekombination in der Hefe durchgeführt wird, an der normalen cDNA-Klonierungsstelle linearisiert wird und Transkriptionspromotor- und -terminatorsequenzen besitzt, wobei die Rekombination an diesen Sequenzen erfolgt.

10. Verfahren nach einem der Ansprüche 1 bis 9, **dadurch gekennzeichnet, dass** der Expressionsvektor außerdem die Fähigkeit besitzt, sich autonom in eukaryotischen Zellen und/oder in *Escherichia coli* zu replizieren.

11. Verfahren nach einem der Ansprüche 1 bis 10, **dadurch gekennzeichnet, dass** der verwendete Hefestamm eine genetische Modifikation aufweist, welche die Überexpression von mindestens einem Protein ermöglicht, das aus der Gruppe ausgewählt ist, bestehend aus einer endogenen oder exogenen P450-Reduktase, einem Adrenodoxin, einer Adrenodoxin-Reduktase, einem heterologen Cytochrom b5, einem Phase-II-Enzym (insbesondere einer Epoxid-Hydrolase).

12. Verfahren nach einem der Ansprüche 1 bis 11, **dadurch gekennzeichnet, dass** auf den Schritt b. folgende Schritte folgen:
c. Extrahieren der Plasmid-DNA von mindestens einem Hefeklon,
d. Transformieren eines *Escherichia coli*-Stammes mit der extrahierten Plasmid-DNA und Selektieren der transformierten Klone auf geeignetem Medium, um eine Unterscheidung von Elementen der kombinatorischen funktionellen Expressionsbank zu erhalten.

13. Verfahren zur Produktion funktioneller aktiver Mosaikproteine, **dadurch gekennzeichnet, dass** man eine kombinatorische funktionelle Expressionsbank durch ein Verfahren nach einem der Ansprüche 1 bis 12 konstruiert, dass man die Mosaikproteine exprimiert und dass man die funktionellen aktiven Mosaikproteine durch Untersuchung ihrer Aktivität selektiert.

14. Verfahren nach Anspruch 13, **dadurch gekennzeichnet, dass** die funktionellen aktiven Mosaikproteine Enzymderivate sind.

15. Verfahren nach Anspruch 14, **dadurch gekennzeichnet, dass** die funktionellen aktiven Mosaikproteine Derivate von P450-Cytochromen sind.

16. Verfahren zur Analyse einer kombinatorischen funktionellen Expressionsbank nach einem der Ansprüche 1 bis 12, **dadurch gekennzeichnet, dass** es folgende Schritte umfasst:
a. Herstellen einer kombinatorischen funktionellen Expressionsbank durch ein Verfahren nach einem der Ansprüche 1-12,
b. Transformation eines *Escherichia coli*-Stammes mit Plasmid-DNA, die aus dem Hefestamm oder einem Hefe-Pool extrahiert wurde,
c. Hybridisierung der Plasmid-DNA, die in den jeweiligen *Escherichia coli*-Einzelklonen enthalten ist, die am Ende von Schritt a. erhalten werden, mit einer oder mehreren Sonde(n), die für eine Parentalsequenz spezifisch ist/sind.

17. Verfahren nach Anspruch 16, **dadurch gekennzeichnet, dass** die Hybridisierung an einem DNA-Makro- oder Mikroarray erfolgt, wobei der Array entweder aus Plasmid-DNA besteht, die in den jeweiligen *Escherichia coli*-Einzelklonen enthalten ist, die am Ende von Schritt a. erhalten werden, oder aus einem PCR-Produkt davon oder aus den spezifischen Sonden, gebunden an einen festen Träger, wobei jede der Nukleinsäuren anhand ihrer Position in dem Array aufgefunden wird.

18. Verfahren zur Bestimmung von Zusammenhängen zwischen Sequenzsignaturen und funktionellen Signaturen eines Proteins, **dadurch gekennzeichnet, dass** es folgende Schritte umfasst:
a. Herstellen einer kombinatorischen funktionellen Expressionsbank durch ein Verfahren nach einem der Ansprüche 1-12,
b. Produzieren aktiver funktioneller Mosaikproteine durch ein Verfahren nach einem der Ansprüche 13 bis 15,
c. Analysieren von funktionellen und/oder Aktivitätsunterschieden zwischen den Mosaikproteinen,
d. Analysieren der Nukleinsäuren, die den Mosaikproteinen entsprechen, durch ein Verfahren nach Anspruch 16 oder 17, gegebenenfalls gefolgt von einem Verfahren zur Analyse eines Hybridisierungsfootprints, umfassend folgende Schritte:
i. Berechnen der Häufigkeit des Auftretens von jeder der Kombinationen, die im Schritt a. erhalten werden,
ii. Durchführen einer statistischen Analyse durch Vergleichen der im Schritt i. berechneten Auftrittshäufigkeit mit der theoretischen Auftrittshäufigkeit für jede Kombination durch eine angemessene mathematische und statistische Behandlung,
e. Bestimmen der Zusammenhänge, die zwischen den im Schritt d. beobachteten Sequenzstrukturen mit den im Schritt c. beobachteten funktionellen und/oder Aktivitätsunterschieden existieren können.

19. Verfahren zur Vorhersage von Strukturen mit einer bestimmten Funktion, **dadurch gekennzeichnet, dass** man das Verfahren nach Anspruch 18 durchführt, um die Sequenzbereiche oder die Zusammenhänge zwischen den Sequenzbereichen zu identifizieren, die mit dieser Funktion zusammenhängen, und **dadurch**, dass man daraus die gesuchte Struktur ableitet.

20. Verfahren zur Gewinnung eines Proteins, das verbesserte Eigenschaften besitzt, **dadurch gekennzeichnet, dass** es folgende Schritte umfasst:
a. Konstruieren einer kombinatorischen funktionellen Expressionsbank durch ein Verfahren nach einem der Ansprüche 1-12,
b. Analysieren der kombinatorischen funktionellen Expressionsbank durch ein Verfahren nach einem der Ansprüche 16 oder 17,
c. Analysieren der im Schritt b. erhaltenen Hybridisierungsfootprints durch ein Verfahren zur Analyse von Hybridisierungsfootprints, umfassend folgende Schritte:
i. Berechnen der Häufigkeit des Auftretens von jeder der möglichen Kombinationen, die im Schritt a. erhalten werden,
ii. Durchführen einer statistischen Analyse durch Vergleichen der im Schritt c.i. berechneten Auftrittshäufigkeit mit der theoretischen Auftrittshäufigkeit für jede Kombination durch eine angemessene mathematische und statistische Behandlung,
d. Bestimmen der Zusammenhänge zwischen den Sequenzstrukturen und den funktionellen Strukturen der Proteine durch Vergleich der Hybridisierungsfootprints mit den Eigenschaften der entsprechenden Mosaikproteine durch ein Verfahren nach Anspruch 18,
e. Vorhersagen der interessierenden Strukturen oder der Organisationen von Strukturen in den Mosaikproteinen durch ein Verfahren nach Anspruch 19,
f. Wiederholen der Schritte a. bis e. unter Verwendung der Nukleinsäuren als Ausgangsnukleinsäuren zur Erzeugung der kombinatorischen funktionellen Expressionsbank, welche die im Schritt e. identifizierten interessierenden Strukturen oder Organisationen von Strukturen tragen, eine ausreichende Anzahl von Malen, um das Protein zu erhalten, das die gesuchten verbesserten Eigenschaften besitzt.

21. Verfahren zur Bestimmung einer Struktur eines wichtigen Proteins als Reaktion auf einen Selektionsdruck ausgehend von einer kombinatorischen funktionellen Expressionsbank, die durch ein Verfahren nach einem der Ansprüche 1 bis 12 erhalten und gemäß einem Verfahren nach einem der Ansprüche 16 oder 17 analysiert wird, für dessen Elemente eine Signatur erhalten wurde, umfassend folgende Schritte:
- Standardisieren der Bank, wobei sichergestellt wird, dass sich jede Signatur mit der gleichen Wahrscheinlichkeit in der standardisierten Bank befindet,
- Anlegen eines Selektionsdrucks,
- Durchführen einer statistischen Analyse der Häufigkeiten der Sequenzsignaturen der so erhaltenen neuen Bank in Bezug auf diejenigen der standardisierten Ausgangsbank,
- Untersuchen der Veränderung der Auftrittshäufigkeiten der Sequenzsignaturen der erhaltenen neuen Bank in Bezug auf die standardisierte Ausgangsbank, indem folglich die Strukturen abgeleitet werden, die als Reaktion auf den Selektionsdruck vorliegen oder fehlen.
